# FASCICULE DE BREVET EUROPEEN

(11) **EP 3 826 671 B1**
(45) Date de publication et mention de la délivrance du brevet: **14.08.2024**
(21) Numéro de dépôt: 19758792.6
(22) Date de dépôt: 12.07.2019
(51) Int. Cl.: A61K 39/12, A61K 39/155, C12N 7/04, C12N 15/86

(54) **NOUVELLE SOUCHE VIRALE ATTENUEE ET SON UTILISATION EN TANT QUE VACCIN**
NEUER ABGESCHWÄCHTER VIRUSSTAMM UND DESSEN VERWENDUNG ALS IMPFSTOFF
NEW ATTENUATED VIRUS STRAIN AND USE THEREOF AS A VACCINE

(30) Priorité: 23.07.2018 FR 1856801; 21.03.2019 FR 1902934
(43) Date de publication de la demande: 02.06.2021
(73) Titulaire: Université Claude Bernard Lyon 1, 69100 Villeurbanne (FR); Centre National de la Recherche Scientifique (CNRS), 75016 Paris (FR); Ecole Normale Supérieure de Lyon, 69007 Lyon (FR); INSTITUT NATIONAL DE LA SANTE ET DE LA RECHERCHE MEDICALE - INSERM, 75013 Paris (FR); Université Laval, Québec, Québec G1V 0A6 (CA)
(72) Inventeur: ROSA-CALATRAVA, Manuel, 69003 Lyon (FR); BOIVIN, Guy, Québec, QC G1V1E7 (CA); DUBOIS, Julia, 69140 Rillieux-la-Pape (FR); PIZZORNO, Mario Andres, 69007 Lyon (FR); TERRIER, Olivier, 69008 Lyon (FR); HAMELIN, Marie-Eve, Québec, QC G2B 0K6 (CA); CAVANAGH, Marie-Hélène, Levis, QC G7A 0B8 (CA)
(74) Mandataire: Regimbeau
(86) Numéro de dépôt international: PCT/FR2019/051759
(87) Numéro de publication internationale: WO 2020/021180

(56) Documents cités:
- WO-A1-2007/038862
- US-A1- 2004 241 188
- BIACCHESI S ET AL: "Recombinant human Metapneumovirus lacking the small hydrophobic SH and/or attachment G glycoprotein: deletion of G yields a promising vaccine candidate", JOURNAL OF VIROLOGY, THE AMERICAN SOCIETY FOR MICROBIOLOGY, US, vol. 78, no. 23, 1 December 2004 (2004-12-01), pages 12877 - 12887, XP002318326, ISSN: 0022-538X, DOI: 10.1128/JVI.78.23.12877-12887.2004
- BIACCHESI STEPHANE ET AL: "Infection of nonhuman primates with recombinant human metapneumovirus lacking the SH, G, or M2-2 protein categorizes each as a nonessential accessory protein and identifies vaccine candidates", JOURNAL OF VIROLOGY, vol. 79, no. 19, October 2005 (2005-10-01), pages 12608 - 12613, XP002790247, ISSN: 0022-538X
- HAMELIN M E ET AL: "Prophylactic and therapeutic benefits of a monoclonal antibody against the fusion protein of human metapneumovirus in a mouse model", ANTIVIRAL RESEARCH, ELSEVIER BV, NL, vol. 88, no. 1, 1 October 2010 (2010-10-01), pages 31 - 37, XP027317844, ISSN: 0166-3542, [retrieved on 20100707]
- CHRISTIAN E PALAVECINO ET AL: "Understanding Lung Immunopathology Caused by the Human Metapneumovirus: Implications for Rational Vaccine Design", CRITICAL REVIEWS IN IMMUNOLOGY., vol. 35, no. 3, 1 January 2015 (2015-01-01), US, pages 1 - 33, XP055532144, ISSN: 1040-8401, DOI: 10.1615/CritRevImmunol.2015013844
- BIACCHESI S ET AL: "Recovery of human metapneumovirus from cDNA: optimization of growth in vitro and expression of additional genes", VIROLOGY, ELSEVIER, AMSTERDAM, NL, vol. 321, no. 2, 10 April 2004 (2004-04-10), pages 247 - 259, XP004499206, ISSN: 0042-6822, DOI: 10.1016/J.VIROL.2003.12.020

## Description

### DOMAINE DE L'INVENTION

La présente invention concerne des souches virales modifiées génétiquement à partir d'une nouvelle souche de *metapneumovirus* humain.

La présente invention concerne ces souches modifiées, dont la virulence est atténuée, pour leur utilisation dans le traitement prophylactique ou thérapeutique des infections aux virus de la famille des *Pneumoviridae,* ainsi que des compositions vaccinales comprenant lesdites souches virales modifiées.

### INTRODUCTION

Les infections aiguës des voies respiratoires inférieures sont l'une des causes majeures de morbidité et de mortalité à l'échelle mondiale. Chez les enfants de moins de 5 ans en particulier, elles représentent jusqu'à plus de 15 % de la mortalité, selon les pays évalués, et sont ainsi la deuxième cause de mortalité d'après l'Organisation Mondiale de la Santé.

La majorité des infections aiguës des voies respiratoires inférieures, associées à des pathologies variables allant du simple rhume à la pneumonie grave, sont notamment causées par des virus, tels que ceux appartenant aux familles suivantes :
(i) virus de la famille des *Orthomyxoviridae* (comprenant notamment les virus Influenza) ;
(ii) virus de la famille des *Paramyxoviridae* ;
(iii) virus de la famille des *Adenoviridae* ;
(iv) virus de la famille des *Picomaviridae* ;
(v) virus de la famille des *Coronaviridae* et
(vi) virus de la famille des *Pneumoviridae,* comprenant notamment le virus respiratoire syncytial humain et le métapneumovirus humain.

Le virus respiratoire syncytial humain (hRSV) et le métapneumovirus humain (hMPV) sont des virus responsables d'infections aiguës des voies respiratoires telles que des bronchiolites, des bronchites ou des pneumonies. Ils touchent principalement les populations à risques que sont les jeunes enfants de moins de 5 ans, les personnes âgées et les personnes immunodéprimées.

Le virus hRSV est l'agent étiologique principal des bronchiolites et pneumonies chez les nourrissons de moins de 1 an, avec une incidence accrue en-dessous de 6 mois de vie. Chez l'adulte, l'infection à hRSV est rare et bénigne, sauf chez le sujet âgé. La transmission se fait essentiellement par voie respiratoire, le virus se répliquant dans le tractus respiratoire.

Le virus hRSV a été isolé en 1957. Il s'agit d'un virus à ARN monocaténaire de polarité négative, de type « enveloppé », à capside à symétrie hélicoïdale. Il appartient à la famille des *Pneumoviridae* et au genre *Orthopneumovirus.*

Le virus hMPV est prévalent dans les bronchiolites et pneumonies chez les nourrissons, et touche particulièrement sévèrement les enfants entre 1 et 3 ans.

Les principaux symptômes de l'infection par le hMPV chez l'enfant comportent la rhinorrhée, la toux, la détresse respiratoire ou encore de la fièvre. Le hMPV peut aussi causer des infections des voies respiratoires supérieures, pouvant être associées à des otites, alors que les symptômes non respiratoires, tels que la diarrhée, les vomissements et l'apparition d'érythème, sont plus rares. Le hMPV cible préférentiellement les cellules ciliées de l'arbre respiratoire humain.

Une infection par hMPV induit des modifications histo-pathologiques dans les poumons de l'organisme hôte, et génère en particulier les effets physiologiques suivants :
- Des dommages au niveau de l'épithélium respiratoire ;
- Une production excessive de mucus, et
- Une inflammation aigüe, notamment au niveau des tissus pulmonaires interstitiels, liée à une sécrétion importante de cytokines pro-inflammatoires et chimiokines.

Dans les pathologies respiratoires chez les adultes, le virus hMPV a été identifié chez 5 à 10% des adultes ou personnes âgées présentant une infection aigüe des voies respiratoires, et chez 3 à 5% des adultes ayant une exacerbation d'une pathologie pulmonaire chronique ou d'une pneumonie acquise en communauté.

Le virus hMPV a été isolé et décrit pour la première fois en 2001 aux Pays-Bas (van den Hoogen *et al.*, 2001). L'année suivante, plusieurs souches de HMPV ont été isolées chez des patients localisés en Amérique du Nord (Peret *et al.*, 2002). Les hMPV sont des virus à ARN monocaténaire négatif, appartenant à la famille des *Pneumoviridae* et au genre *Metapneumovirus.*

L'âge moyen des enfants hospitalisés des suites d'une infection par le hMPV est de 6 à 12 mois, soit plus tard que celle provoquée par le hRSV, qui survient principalement entre 0 et 3 mois. Toutefois, l'épidémiologie du hMPV présente de nombreux points communs avec celle du hRSV :
- il est présent sur tous les continents ;
- sa distribution annuelle est majoritairement hivernale et printanière, recoupant celle du hRSV ;
- sa transmission se fait aussi probablement par gouttelettes salivaires.

Il n'existe aujourd'hui sur le marché aucune modalité prophylactique ou thérapeutique efficace et spécifique contre l'infection par les virus hRSV et hMPV, bien que d'actives recherches soient en cours (Mazur *et al.*, 2018).

A titre de traitement, la ribavirine, non exempte d'effets indésirables, ou encore les immunoglobulines intraveineuses, très onéreuses, peuvent être utilisées ponctuellement dans les cas graves d'infections par hMPV ou hRSV. D'autres types de traitements sont en cours de développement tels que l'utilisation de peptides inhibiteurs de fusion, les glycosaminoglycanes sulfate, les ARN interférents et certains immunomodulateurs.

La démarche clinique usuelle et largement privilégiée aujourd'hui consiste à traiter surtout les symptômes de l'infection, en mettant les patients sous assistance respiratoire (administration d'oxygène ou ventilation mécanisée) et en leur administrant des bronchodilatateurs, corticostéroïdes et/ou antibiotiques pour prévenir ou traiter les surinfections bactériennes.

En ce qui concerne la vaccination, les populations particulièrement touchées par le hMPV étant les nourrissons, les jeunes enfants et les personnes âgées, il est crucial de pouvoir disposer rapidement de vaccins efficaces et sécuritaires, afin de réduire les atteintes respiratoires sévères qui ont un impact dramatique dans ces tranches d'âge.

La mise au point d'un vaccin contre le hRSV et le hMPV représente donc non seulement un enjeu sanitaire majeur, mais également un réel enjeu socio-économique avec l'objectif de réduire les coûts importants des traitements et d'hospitalisations associés à ces infections, et de diminuer l'utilisation d'antibiotiques dans le contexte des surinfections bactériennes, et ainsi de limiter l'émergence de résistances.

### ETAT DE LA TECHNIQUE

Les tableaux 1, 2 et 3 ci-dessous listent les différentes approches en cours de développement pour obtenir des vaccins vivants atténués à partir de souches virales sauvages de hMPV.

Des souches virales sont considérées comme étant *atténuées in vitro* lorsque celles-ci présentent une capacité réplicative diminuée par rapport au virus sauvage (WT), et/ou lorsque ces souches virales entrainent la formation de foyers infectieux, notamment de syncytia (cellules adjacentes fusionnant suite à l'infection virale), plus restreints. *In vivo*, les souches virales atténuées se répliquent à un titre maximal plus faible et/ou induisent une pathologie moins sévère (en termes de perte de poids ou de profil inflammatoire ou d'atteintes histo-pathologiques) que la souche virale sauvage.

**Tableau 1. Liste des candidats vaccins vivants atténués sur la base d'une souche de virus hMPV présentant une ou des mutations, développés ou en cours de développement**

| **Nom du virus** | **Description** | **Atténuation *in vitro*** | **Atténuation *in vivo*** | **Anticorps neutralisants protecteurs** | **Référence** |
|---|---|---|---|---|---|
| cp-HMPV M11 (B1/NL/1/99) | Insertion de 11 mutations aa parmi les 17 induites par des passages sur cellules Vero, température décroissante jusqu'à 25°C. | ✔ Vero > 39°C | ✔ H | ✔ H | (Herfst, de Graaf et al. 2008) |
| cp-HMPV HRSV3 (B1/NL/1/99) | 4 mutations *cp (cold passaging)* de hRSV | ✔ Vero > 37°C | ✔ H | ✔ H | (Herfst, de Graaf et al. 2008) |
| rhMPV-R329K rhMPV-D331A (A1/NL/1/00) | Mutations dans le domaine de liaison à l'intégrine de la protéine F | ✔ LLC-MK2 | ✔ CR | ✔ CR | (Wei, Zhang et al. 2014) |
| HMPV M2 (A1/NL/1/00) | Suppression du site de N-glycosylation (aa 172) de la protéine F | ✔ Vero. | ✔ M | ✔ M | (Yu, Li et al. 2012) (Liu, Shu et al. 2013) |
| | | | ✔ M SCID | | |
| HMPV-MTase (A1/NL/1/00) | Mutations du site méthyltransférase de la polymérase L G1696A, G1700A, and D1755A | ✔ LLC-MK2 | ✔ CR | ✔ CR | (Zhang, Wei et al. 2014) |

Le terme « Vero » désigne une lignée de cellules de rein de singe vert africain, couramment utilisée en culture cellulaire pour tester divers virus.

Le terme « LLC-MK2 » désigne une lignée cellulaire issue de cellules de rein de singe rhésus, utilisée pour des tests d'infection par divers virus.

Le symbole ✔ indique que les cellules utilisées sont susceptibles à l'infection virale.

Les abréviations suivantes sont utilisées pour les modèles d'études *in vivo* : M pour *Mouse*; H pour hamster; CR pour *Cotton Rat*; CM pour Cynomolgus Macaque; AGM pour *African Green Monkey;* Ch pour Chimpanzé; Rh pour Singe Rhésus; et SCID pour *Severe Combined ImmunoDeficiency.*

**Tableau 2. Liste des candidats vaccins vivants atténués développés ou en développement, comprenant une souche de virus hMPV présentant une délétion complète d'au moins un gène**

| **Nom du virus** | **Description** | **Atténuation *in vitro*** | **Atténuation *in vivo*** | **Anticorps neutralisants protecteurs** | **Références** |
|---|---|---|---|---|---|
| HMPV-ΔSH (A2/CAN97-83) | Délétion du gène SH | ∅ LLC-MK2 | ∅ H | ✔ H | (Biacchesi, Skiadopoulos et al. 2004) (Biacchesi, Pham et al. 2005) |
| | | | ✔ Ch | ✔ Ch | |
| HMPV-ΔG (A2/CAN97-83) | Délétion du gène G | ∅ LLC-MK2 | ✔ H à 3 jours | ✔ H | (Biacchesi, Skiadopoulos et al. 2004) (Biacchesi, Pham et al. 2005) |
| | | | ∅ H > 3 jours | | |
| | | | ✔ Ch | ✔ Ch | |
| HMPV- ΔSH/ΔG (A2/CAN97-83) | Délétion des gènes SH et G | ∅ LLC-MK2 | ✔ H à 3 jours. | ✔ H | (Biacchesi, Skiadopoulos et al. 2004) |
| | | | ∅ H > 3 jours | | |
| HMPV- ΔM2-2 (A2/CAN97-83) | Mutations codon initiation + codon stop | ∅ Vero | ✔ H | ✔ H | (Buchholz, Biacchesi et al. 2005) (Biacchesi, Pham et al. 2005) (Schickli, Kaur et al. 2008) |
| | | | ✔ Ch | ✔ Ch | |
| HMPV- ΔM2-1 (A2/CAN97-83) | Mutation codon stop | ∅ Vero | ∅ H | ∅ H | (Buchholz, Biacchesi et al. 2005) |
| HMPV- ΔM2-1/ ΔM2-2 (A2/CAN97-83) | Délétion du gène M2 complet | ∅ Vero | ∅ H | ∅ H | (Buchholz, Biacchesi et al. 2005) |

| | | | | | |
|---|---|---|---|---|---|
| Δ : délétion totale du gène. Ø = pas d'atténuation ou d'avantage réplicatif. | | | | | |

Toutes les souches virales atténuées testées sont dérivées de la souche sauvage CAN97-83 du sous-groupe A2.

**Tableau 3. Liste des candidats vaccins vivants atténués développés ou en développement, basés sur une souche de virus hMPV chimérique**

| **Nom du virus** | **Description** | **Atténuation *in vitro*** | **Atténuation *in vivo*** | **Ac neutralisants protecteurs** | **Test Clinique** | **Réf.** |
|---|---|---|---|---|---|---|
| HMPV-Pa (A2/CAN97-83) | Fond | ∅ Vero | ✔ H | ✔ H | Phase I | (Pham, Biacchesi et al. 2005) (Karron, San Mateo et al. 2017) |
| | génétique virus HMPV (SH stabilisé) Échange du gène P avec l'homologue aMPV C | | ✔ AGM | ✔ AGM | NCT01255410 | |
| HMPV Na (A2/CAN97-83) | Fond génétique virus | ∅ Vero | ✔ H à 3 jours. | ✔ H | | (Pham, Biacchesi et al. 2005) |
| | HMPV (SH stabilisé) | | Ø H > 3 jours | ✔ AGM | | |
| | Échange du gène N avec l'homologue aMPV C | | ✔ AGM | | | |
| b/hPIV3/ hMPV F2 (A1/NL/1/00) | Fond | - | ∅ H | ✔ H | | (Tang, Schickli et al. 2003) (Tang, Mahmood et al. 2005) |
| | génétique PIV- | | ∅ AGM | ✔ AGM (hMPV/hPIV) | | |
| | 3 bovin Echange des gènes F et HN avec leur homologue hPIV-3 Ajout F hMPV en 2^{ème} position sur le génome | | ✔ Rh séronégatif | | | |
| SeV-MPV-Ft (A2/ CAN00-16) | Fond génétique virus SeV Ajout gène F hMPV tronqué pour son domaine TM | - | ✔ CR | ✔ CR | | (Russell, Jones et al. 2017) |

Les abréviations suivantes sont utilisées : TM = domaine transmembranaire ; PIV = Virus Parainfluenza; SeV = Virus Sendai.

La demande internationale WO 2005/014626 est relative à plusieurs souches de virus hMPV, désignées par les dénominations suivantes : CAN 97-83, CAN 98-75 and HMPV 00-1. Cette demande décrit une souche de virus hMPV recombinante, désignée CAN 97-83, modifiée génétiquement pour atténuer sa virulence. Les modifications proposées concernent notamment la délétion totale des gènes codant pour les protéines G et/ou SH. Les souches virales modifiées peuvent être utilisées en thérapie humaine, pour prévenir ou traiter les infections aux pneumovirus. L'administration de la souche sauvage ou de ces souches atténuées à des hamsters permet de les protéger contre une infection ultérieure par un virus HMPV CAN 97-83. Toutefois, WO 2005/014626 ne fait pas état de propriétés complètes de protection, en particulier aucun suivi de poids des animaux traités n'est réalisé. Les mêmes résultats expérimentaux sont présentés dans les articles scientifiques cités dans le tableau 2.

Avec les mêmes souches atténuées, des résultats *in vivo* ont ensuite été obtenus sur un modèle animal de singes ; les souches virales recombinantes délétées des gènes SH, G et M2-2 continuent à se répliquer dans les voies respiratoires des singes ; et par ailleurs induisent la production d'anticorps neutralisants tout comme la souche virale sauvage CAN97-83.

D'autres souches de hMPV isolées ont été décrites dans le brevet US 8,841 ,433, leur utilisation pour la préparation de vaccins étant également proposée.

Les vaccins basés sur l'utilisation de souches virales vivantes atténuées présentent de nombreux avantages.

D'une part, les vaccins produits à partir de virus vivants atténués présentent l'avantage d'induire une forte réponse immunitaire à partir d'un virus dont la capacité à se multiplier est considérablement réduite, permettant ainsi d'éviter l'apparition de la pathologie tout en mimant l'infection naturelle. Lors de la mise au point de ces souches atténuées, il est important de trouver la balance idéale entre l'atténuation et l'immunogénicité.

D'autre part, ces vaccins peuvent être administrés par voie intra-nasale, et ainsi mimer la voie d'entrée naturelle des virus sauvages, induisant ainsi une réponse immunitaire assez similaire à la réponse physiologique à une infection.

De plus, cette stratégie de vaccination ne génère pas une réaction inflammatoire exagérée, comme cela peut être le cas avec les vaccins inactivés. Enfin, l'ajout d'adjuvant n'est généralement pas nécessaire.

C'est donc la stratégie vaccinale optimale pour la prévention dans les populations à risque tels que les jeunes enfants et les nourrissons.

### EXPOSE DE L'INVENTION

L'invention a pour objet une souche virale atténuée dérivée d'une souche clinique particulière rC-85473 de *metapneumovirus* humain, comprenant la séquence génomique représentée par la séquence SEQ ID NO. 1, ladite souche atténuée comprenant une ou des modifications génétiques de ladite séquence SEQ ID NO.1 atténuant la virulence de ladite souche, caractérisée en ce que les modifications de la séquence SEQ ID NO.1 consistent en l'inactivation du gène codant pour la protéine SH et/ou l'inactivation du gène codant pour la protéine G.

Les souches virales atténuées selon l'invention sont les souches recombinantes modifiée par inactivation ou délétion d'au moins un gène codant pour l'une des protéines accessoires G et SH, pour générer des virus recombinants désignés ΔG rC-85473 et ΔSH rC-85473, respectivement.

La présente invention a pour objet une souche virale atténuée dérivée d'une souche clinique de *metapneumovirus* humain, comprenant la séquence génomique représentée par la séquence SEQ ID NO. 1, ladite souche atténuée comprenant au moins une modification génétique choisie parmi : l'inactivation du gène codant pour la protéine accessoire G, et l'inactivation du gène codant pour la protéine accessoire SH.

Ces souches ont été caractérisées aux niveaux de leur infectivité et leur réplication virale en modèles cellulaires *in vitro* (lignée cellulaire LLC-MK2) et ex *vivo* (épithéliums respiratoires humains reconstitués et cultivés à l'interface air-liquide), ainsi qu'au niveau de la pathogénèse virale et de leur propriété de protection vaccinale en modèle murin d'infection par hMPV.

Les résultats présentés dans la section expérimentale mettent en évidence les propriétés spécifiques de ces souches virales ΔG rC-85473 et ΔSH rC-85473, en comparaison de celles de souches virales modifiées de manière similaire, également délétées des gènes G ou SH, mais dérivées d'une autre souche clinique de virus hMPV (CAN98-75).

Les virus ΔG ou ΔSH rC-85473 présentent en particulier :
(i) un caractère viral atténué en modèle murin, à la différence du virus recombinant ΔSH CAN98-75 qui lui est très virulent ;
(ii) tout en conservant leur capacité de réplication en systèmes cellulaires adhérents (lignée de référence LLC-MK2) et en suspension à la différence des virus recombinants CAN98-75 qui sont beaucoup moins productifs ;
(iii) Les virus ΔG ou ΔSH C-85473 présentent de manière avantageuse des propriétés de protection significatives : 100% de survie des souris infectées, peu de perte de poids, induction d'une réponse d'anticorps neutralisants contre la souche virale homologue (C-85473, sérotype A) et au moins contre une souche hétérologue (CAN98-75, sérotype B), ainsi qu'une pathogénèse pulmonaire réduite chez les animaux infectés, vis-à-vis d'un challenge infectieux par un virus hMPV sauvage (souche rC-85473), qui lui occasionne 50% de létalité dans les groupes témoins non immunisés.

Cette souche virale atténuée peut être utilisée *in vivo, ex vivo* et *in vitro* en tant que vecteur d'expression d'au moins un gène exogène, en particulier un gène codant pour un antigène viral, et en premier lieu, des antigènes viraux issus du virus respiratoire syncytial (hRSV) et en particulier sa protéine de fusion F.

L'invention concerne également cette souche virale atténuée pour son utilisation en tant que médicament, et plus particulièrement pour prévenir et/ou traiter les infections par des virus de la famille des *Pneumoviridae.*

Enfin, l'invention concerne également une composition vaccinale comprenant, dans un véhicule pharmaceutiquement acceptable, au moins une souche virale atténuée telle que définie ci-dessus, et optionnellement un adjuvant.

### DESCRIPTION DES FIGURES

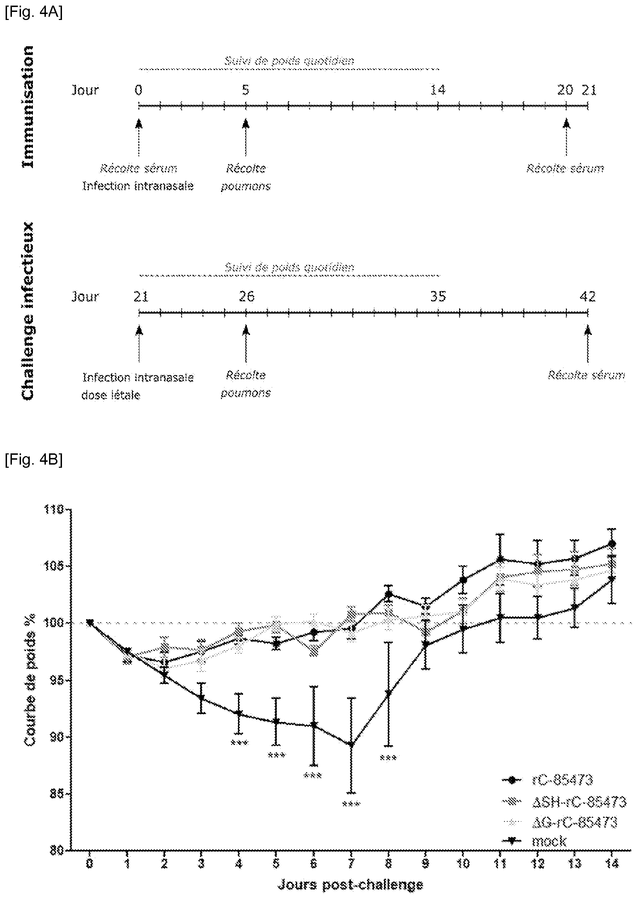

**Figure 1****. Virus recombinants HMPV générés à partir des souches cliniques C-85473 et CAN98-75.**

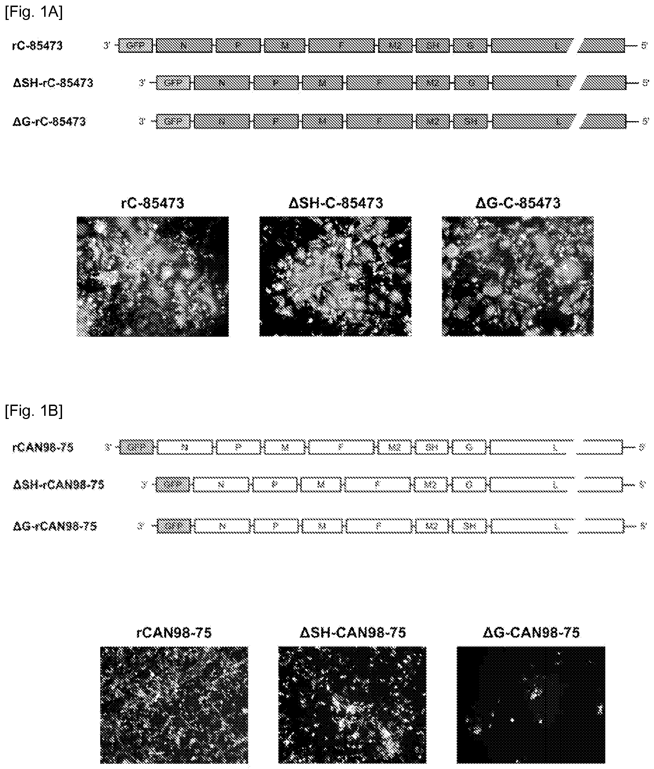

   **[****Fig. 1A****]** Représentation schématique du génome des virus recombinants générés par génétique inverse à partir de la souche clinique C-85473, et dans laquelle a été clonée la séquence codante de la GFP (Green *Fluorescent Protein*) à son extrémité 3'. Le virus rC-85473 contient le génome de la souche C-85473 sauvage exprimant en plus le gène GFP. Les génomes des virus recombinants issus de la souche C-85473 et délétés des gènes SH (ΔSH-rC-85473) ou G (ΔG-rC-85473) sont représentés en dessous. Les virus modifiés génétiquement sont fonctionnels et réplicatifs, tel qu'indiqué par les clichés de cellules LLC-MK2 infectées à une *Multiplicity of Infection* (MOI) de 0,01 par ces virus, et observées en microscopie à fluorescence à trois jours post-infection.
   **[****Fig. 1B****]** Représentation schématique du génome des virus recombinants générés par génétique inverse à partir de la souche clinique CAN98-75, et dans laquelle a été clonée la séquence codante de la GFP (Green *Fluorescent Protein*)*.* Le virus rCAN98-75 contient le génome de la souche CAN98-75 sauvage exprimant en plus le gène GFP à son extrémité 3'. Les génomes des virus recombinants issus de la souche CAN98-75 et délétés des gènes SH (ΔSH-rCAN98-75) ou G (ΔG-rCAN98-75) sont représentés en dessous. Les virus modifiés génétiquement sont fonctionnels et réplicatifs, tel qu'indiqué par les clichés de cellules LLC-MK2 infectées à MOI 0,01 par ces virus et observés en microscopie à fluorescence à trois jours post-infection.
**Figure 2****. Capacités réplicatives *in vitro* des souches virales rC-85473 et rCAN98-75 et de leurs souches dérivées.**
   **[****Fig. 2A****] Titre viral évalué en TCID50/ml dans les surnageants cellulaires** prélevés chaque jour pendant 7 jours après une infection de cellules LLC-MK2 par les virus recombinants rCAN98-75 (ronds noirs), ΔSH-rCAN98-75 (carrés noirs) et ΔG-rCAN98-75 (triangles noirs) à une multiplicité d'infection (MOI) de 0.01.
   **[****Fig. 2B****] Titre viral évalué en TCID50/ml dans les surnageants cellulaires** prélevés chaque jour pendant 7 jours après une infection de cellules LLC-MK2 par les virus recombinants rC-85473 (ronds noirs), ΔSH-rC-85473 (carrés noirs) et ΔG- rC-85473 (triangles noirs) à une multiplicité d'infection de 0.01.
   ** p < 0.01 and ***, p < 0.001 (Tests statistiques Two-way ANOVA).
**Figure 3** : **Caractérisation *in vivo* des virus hMPV recombinants rCAN98-75 et rC-85473 par infection de souris BALB/c** : **courbes de poids, et titres viraux pulmonaires à 5 jours post infection.**
   **[****Fig. 3A****]** Des souris BALB/c ont été infectées par instillation intranasale avec du milieu de culture OptiMem (témoin négatif « mock », triangles noirs) ou 2.5×10⁵ FFU des virus recombinants rCAN98-75 (ronds noirs), ΔSH-rCAN98-75 (carrés gris) et ΔG-rCAN98-75 (triangles gris). Un suivi quotidien de poids et de survie des souris a été effectué pendant 14 jours.
   **[****Fig. 3B****]** 5 jours après l'infection, 5 souris par groupe (sauf groupe témoin négatif) ont été euthanasiées pour une mesure de titres viraux infectieux pulmonaires en TCID50/gramme de poumon.
   **[****Fig. 3C****]** Des souris BALB/c ont été infectées par instillation intranasale avec du milieu de culture OptiMem (témoin négatif « mock », losanges noirs) ou 5×10⁵ TCID50 des virus recombinants rC-85473 (ronds noirs), ΔSH-rC-85473 (carrés gris) et ΔG-rC-85473 (triangles gris). Un suivi quotidien de poids et de survie des souris a été effectué pendant 14 jours.
   **[****Fig. 3D****]** 5 jours après l'infection, 5 souris par groupe (sauf groupe témoin négatif) ont été euthanasiées pour une mesure de titres viraux infectieux pulmonaires en TCID50/gramme de poumon.
   Tests statistiques : Two-way ANOVA pour comparer chaque virus à la condition « mock ». *,p < 0.05, ** p < 0.01 and ***, p < 0.001.
**Figure 4** : **Propriétés protectrices *in vivo* des virus hMPV recombinants ΔG-rC-85473 et ΔSH-rC-85473 en modèle murin BALB/c de challenge infectieux.**
   **[****Fig. 4A****]** Schéma du protocole d'immunisation (en haut) suivi du protocole de challenge infectieux (en bas). La dose létale correspond à 1×10⁶ TCID⁵⁰.
   **[****Fig. 4B****]** Challenge infectieux des souris immunisées avec les souches virales rC-85473 (ronds noirs), ΔSH-rC-85473 (carrés gris) ou ΔG-rC-85473 (triangles gris), ou non immunisées (« mock », triangles noirs)
      21 jours après l'immunisation, les souris ont été infectées par instillation intranasale d'une dose létale (1×10⁶ TCID⁵⁰) du virus rC-85473 sauvage. Un suivi quotidien de poids a été effectué pendant 14 jours.
   **[****Fig. 4C****]** Courbes de survie post-challenge des souris des différents groupes de souris immunisées
      Les souris non immunisées « mock » présentent seulement 50% de survie ; celles immunisées par les virus recombinants rC-85473, ΔSH-rC-85473 ou ΔG-rC-85473 présentent 100% de survie.
   **[****Fig. 4D****]** 5 jours après le challenge infectieux (soit au jour 26 post-immunisation), 4 souris de chaque groupe ont été euthanasiées pour mesurer leurs titres viraux infectieux pulmonaires en TCID50/g de poumons. « nd » signifie non détectable.
   **[****Fig. 4E****]** 5 jours après le challenge infectieux (soit au jour 26 post-immunisation), 4 souris de chaque groupe ont été euthanasiées pour mesurer leurs titres viraux infectieux pulmonaires en RTqPCR (gène N).
   Tests statistiques : Two-way ANOVA pour comparer chaque virus à la condition mock. *,p < 0.05, ** p < 0.01 and ***, p < 0.001.
**Figure 5****: Capacités d'attachement cellulaire *in vitro* des virus recombinants rC-85473 et rCAN98-75 et de leurs versions délétées.**
   **[****Fig. 5A****]** Pourcentage de cellules infectées LLC-MK2 en fonction du temps d'attachement, avec le virus rCAN98-75 (ronds) et ses formes atténuées ΔSH-rCAN98-75 (carrés) et ΔG-rCAN98-75 (triangles).
   **[****Fig. 5B****]** Pourcentage de cellules infectées LLC-MK2 en fonction du temps d'attachement, avec le virus rC-85473 (ronds) et ses formes atténuées ΔSH-rC-85473 (carrés) et ΔG-rC-85473 (triangles).
**Figure 6** **: Capacités réplicatives *ex vivo* des virus recombinants rC-85473 et rCAN98-75 sur un modèle 3D d'épithélium respiratoire humain reconstitué et cultivé à l'interface air-liquide.**
   **[****Fig. 6A****]** Les modèles d'épithéliums respiratoires humains en culture ont été infectés avec les virus recombinants rCAN98-75, ΔSH-rCAN98-75 et ΔG-rCAN98-75 (photos de la ligne supérieure) ou avec les virus recombinants rC-85473 (en blanc), ΔSH- rC-85473 (en gris foncé) et ΔG- rC-85473 (non déterminable) (photos de la ligne inférieure).
   **[****Fig. 6B****]** Quantification de progéniture virale mesurée (via la quantification de génome viral) à la surface apicale des épithéliums infectés avec les virus recombinants rCAN98-75 (WT, en blanc), ΔSH-rCAN98-75 (en gris foncé) et ΔG-rCAN98-75 (en gris clair) (à gauche de la ligne en pointillé) ou avec les virus recombinants rC-85473 (WT), ΔSH- rC-85473 et ΔG- rC-85473 (à droite de la ligne en pointillé).
   **[****Fig. 6C****]** Quantification du génome viral présent au sein des épithéliums infectés avec les virus recombinants rCAN98-75 (WT, en blanc), ΔSH-rCAN98-75 et ΔG-rCAN98-75 (à gauche de la ligne en pointillé) ou avec les virus recombinants rC-85473 (WT, en blanc), ΔSH- rC-85473 et ΔG- rC-85473 (à droite de la ligne en pointillé).
**Figure 7****. Sécrétion de cytokines et chimiokines induites par l'infection des virus recombinants rC-85473 sur un modèle 3D d'épithélium respiratoire humain reconstitué et cultivé à l'interface air-liquide.**
   Des épithéliums respiratoires humains reconstitués (MucilAir^{®} HAE, Epithelix) ont été infectés avec les virus recombinants rC-85473, ΔSH-rC-85473 et ΔG-rC-85473 à une MOI de 0.1. Après 5 jours d'infection, la quantité de plusieurs cytokines et chimiokines est mesurée par la technique Luminex (BioPlex Pro kit assay, BioRad) à partir du milieu basal des épithéliums infectés :

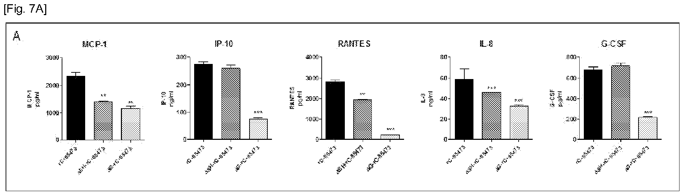

   **[****Fig. 7A****]** Chimiokines MCP-1, IP-10, RANTES, IL-8, G-CSF attractantes de lymphocytes T, monocytes/ macrophages, éosinophiles et/ou neutrophiles.

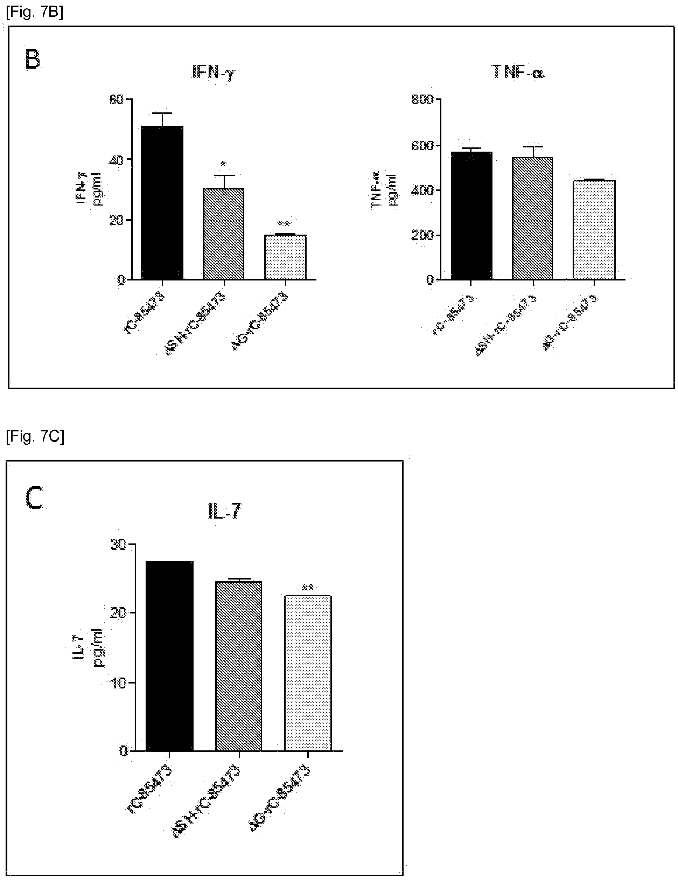

   **[****Fig. 7B****]** Quantification de Cytokines pro-inflammatoires IFN-y et TNF-α.
   **[****Fig. 7C****]** Quantification de Cytokine IL-7, stimulant la différenciation des lymphocytes T et B.

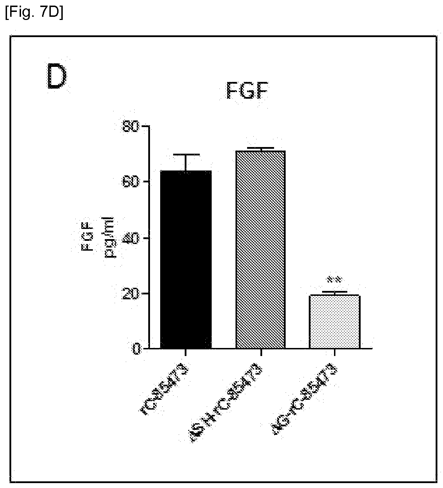

   **[****Fig. 7D****]** Facteur de croissance FGF, impliqué dans la réparation des tissus.
   Tests statistiques : One-way ANOVA pour comparer les virus ΔSH-rC-85473 et ΔG-rC-85473 au virus rC-85473. *, p < 0.05, ** p < 0.01 et ***, p < 0.001.

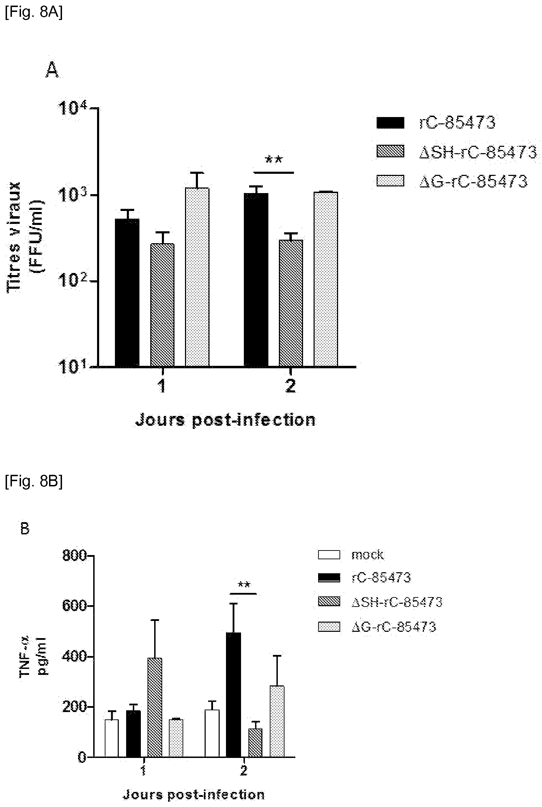

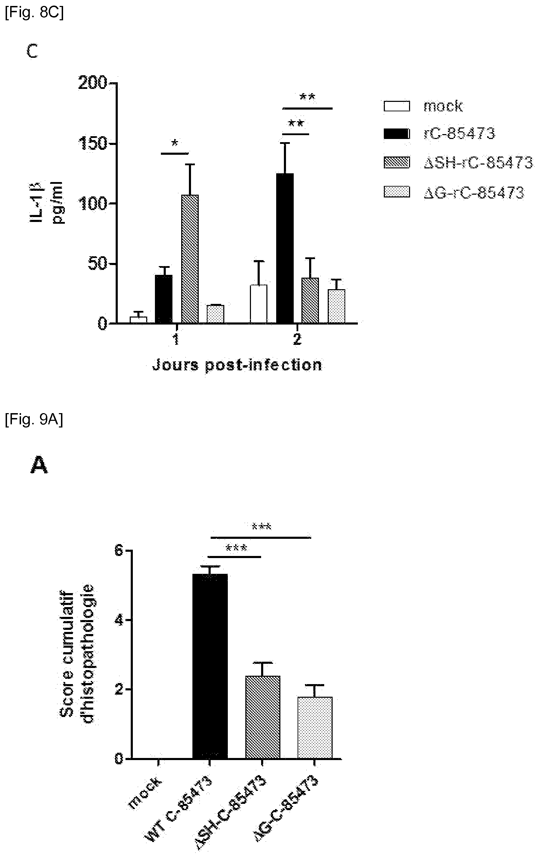

**Figure 8****. Sécrétion viro-induite *in vitro* des cytokines pro-inflammatoires IL-1β et TNF-α en modèle de macrophages murins immortalisés infectés par des virus hMPV recombinants rC-85473.**
   **[****Fig. 8A****]** Titre viral évalué en FFU/ml dans les surnageants cellulaires prélevés chaque jour pendant les 2 jours suivant l'infection d'une lignée cellulaire de macrophages murins immortalisés (lignée BMDM dérivée de moelle osseuse de souris sauvages),par les virus recombinants rC-85473 (noir), ΔSH-rC-85473 (gris foncé) ou ΔG-rC-85473 (gris clair) à une MOI de 0.1.
   **[****Fig. 8B****]** Quantité de la cytokine pro-inflammatoire TNF-α évaluée par test ELISA à partir des surnageants cellulaires prélevés chaque jour pendant les 2 jours suivant l'infection de macrophages murins immortalisés, par les virus recombinants rC-85473 (noir), ΔSH-rC-85473 (gris foncé) ou ΔG-rC-85473 (gris clair) à une MOI de 0.1, en comparaison de macrophages murins immortalisés non-infectés « mock » (blanc).
   **[****Fig. 8C****]** Quantité de la cytokine pro-inflammatoire IL-1β évaluée par test ELISA à partir des surnageants cellulaires prélevés chaque jour pendant les 2 jours suivant l'infection de macrophages murins immortalisés par les virus recombinants rC-85473 (noir), ΔSH-rC-85473 (gris foncé) ou ΔG-rC-85473 (gris clair) à une MOI de 0.1, en comparaison de macrophages murins immortalisés non-infectés « mock » (blanc).
   Tests statistiques : Two-way ANOVA pour comparer les virus ΔSH-rC-85473 et ΔG-rC-85473 au virus rC-85473. *, p < 0.05 et ** p < 0.01.

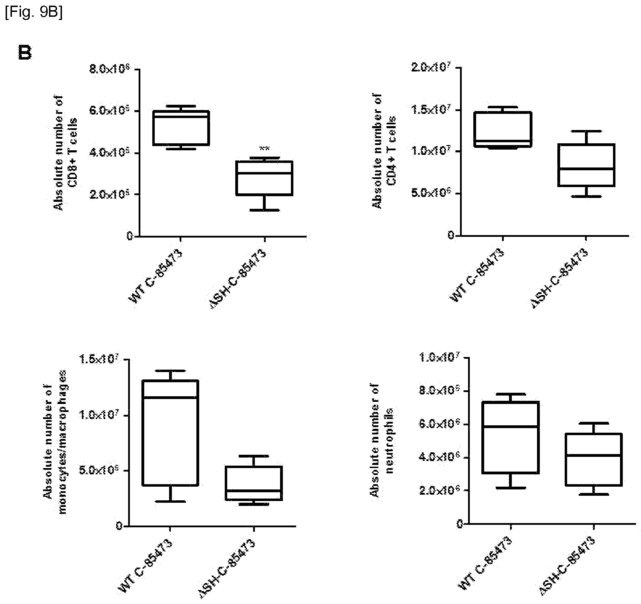

**Figure 9****. Inflammation pulmonaire et recrutement de cellules immunitaires sur le site de l'infection *in vivo*, 5 jours après l'infection de souris BALB/c par les virus hMPV recombinants rC-85473, ΔSH-rC-85473 et ΔG-rC-85473 (inflammation pulmonaire uniquement pour le ΔG-rC-85473).**
   **[****Fig. 9A****]** Score cumulatif d'histopathologie pulmonaire, calculé d'après une évaluation de l'intensité de l'inflammation des tissus bronchial/endobronchial, péribronchial, périvasculaire, interstitiel, pleural et intra-alvéolaire de poumons de souris BALB/c infectées par le virus rC-85473 (noir), ΔSH-rC-85473 (gris foncé), ΔG-rC-85473 (gris clair) ou non infectées « mock », lesdits tissus étant récoltés et fixés au formaldéhyde 5 jours après l'infection virale.
   **[****Fig. 9B****]** Phénotypage des cellules immunitaires déterminé par immuno-marquage (anti-CD45, anti-CD11b, anti-CD170, anti-Ly6C, anti-Ly6G, anti-CD11c, anti-CD115, anti-B220, anti-CD3ε, anti-CD4, anti-CD8a) en cytométrie de flux, à partir de lysats de poumons de souris BALB/c infectées par les virus rC-85473 et ΔSH-rC-85473, après 5 jours d'infection. Les cellules immunitaires recrutées sont classées en quatre catégories : lymphocytes T CD8+ (CD8+ T cells), lymphocytes T CD4+ (CD4+ T cells), monocytes/macrophages, et neutrophiles.
      Tests statistiques : One-way ANOVA pour comparer les virus ΔSH-rC-85473 et ΔG-rC-85473 au virus rC-85473. ** p < 0.01 et ***, p < 0.001.

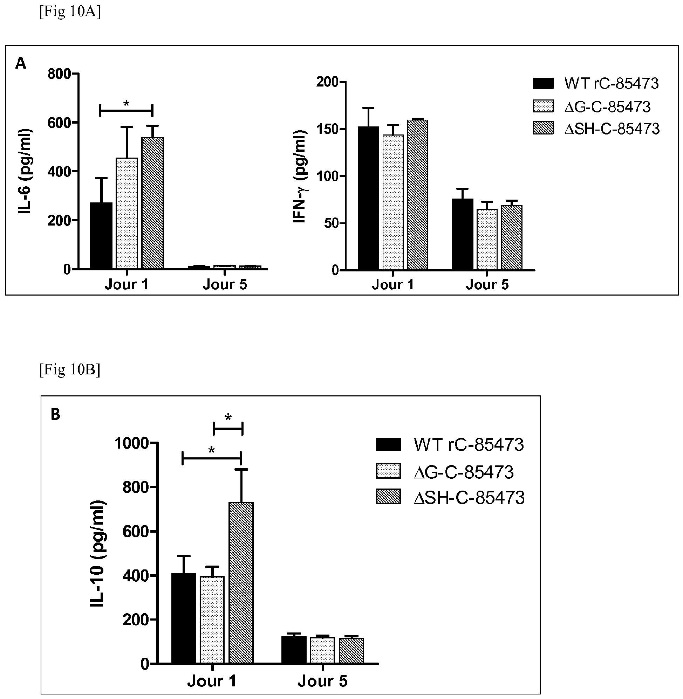

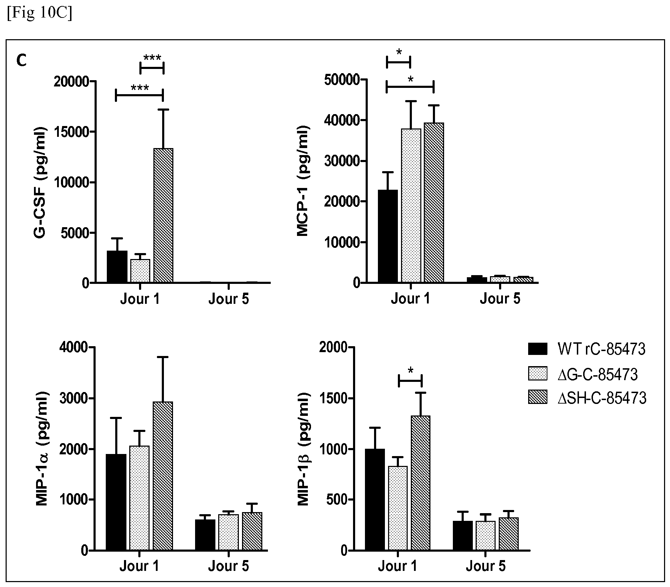

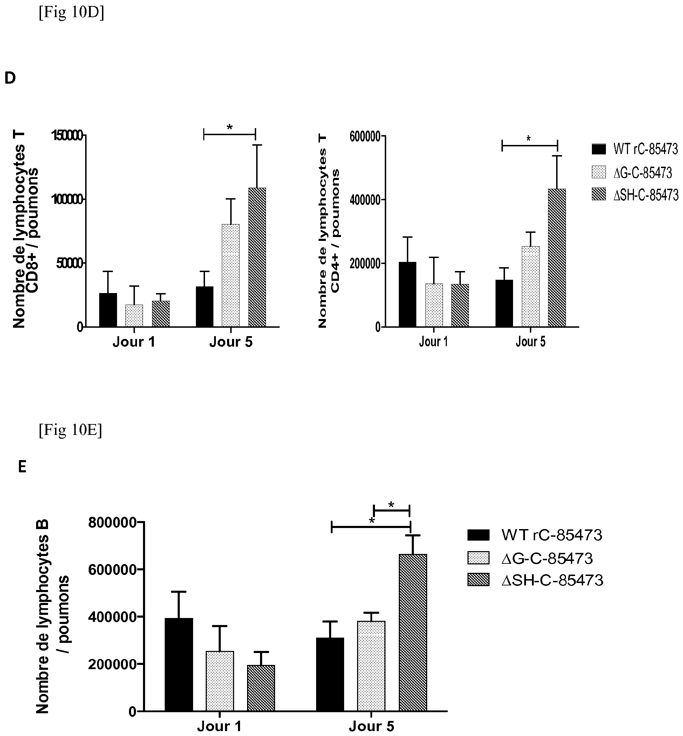

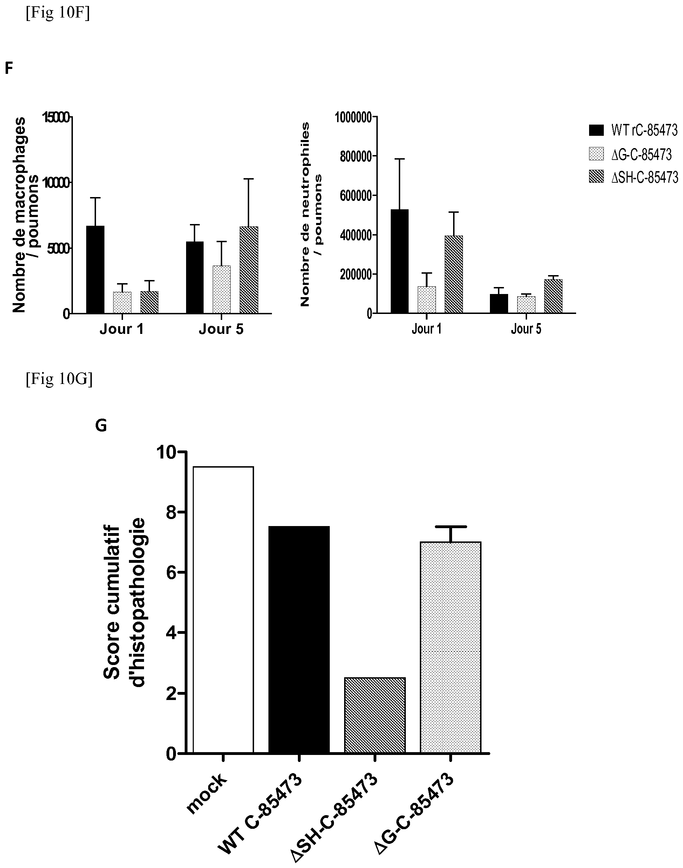

**Figure 10****. Caractérisation de la réponse immunitaire secondaire *in vivo en* modèle murin BALB/c immunisé par les virus hMPV recombinants ΔG-C-85473 et ΔSH-C-85473, suite à un challenge infectieux.**
   1 et/ou 5 jours après le challenge infectieux par le virus WT rC-85473 tel que décrit en Figure 4A, la quantité de plusieurs cytokines et chimiokines est mesurée par la technique Luminex (BioPlex Pro kit assay, BioRad) à partir de lysat de poumons [Fig. 10A-C] ; le phénotypage des cellules immunitaires infiltrées dans le tissu pulmonaire est déterminé par immuno-marquage (anti-CD45, anti-Ly6G, anti-CD11b, anti-CD170/Siglec-F, anti-Ly6C, anti-CD11c, anti-F4/80, anti-B220, anti-CD3ε, anti-CD4 et anti-CD8a) en cytométrie de flux, à partir de lysats de poumons [Fig. 10D-F] ; et le score cumulatif d'histopathologie pulmonaire est calculé d'après une évaluation de l'intensité de l'inflammation des tissus bronchial/endobronchial, péribronchial, périvasculaire, interstitiel, pleural et intra-alvéolaire de poumons de souris fixés au formaldéhyde [Fig. 10G] .
   Dans les figures 10A à 10G, sont représentés :
      - en noir, le groupe de souris BALB/c ayant été immunisé par le virus rC-85473 ;
      - en gris foncé le groupe immunisé par le virus ΔSH-C-85473 ;
      - en gris clair le groupe immunisé par le virus ΔG-C-85473 ;
      - et, si représenté, en blanc le groupe non immunisé dit « mock ».
   N = 2 ou 3 souris / groupe, respectivement pour les analyses histopathologiques ou de sécrétion de cytokines/recrutement cellulaire.
   Tests statistiques : Two-way ANOVA pour comparer chaque groupe les uns aux autres. *,p < 0.05, ** p < 0.01 and ***, p < 0.001.

   **[****Fig. 10A****]** Quantification des cytokines pro-inflammatoires IL-6 et IFN-γ, après 1 et 5 jours de challenge infectieux.
   **[****Fig. 10B****]** Quantification de la cytokine anti-inflammatoire IL-10, après 1 et 5 jours de challenge infectieux.
   **[****Fig. 10C****]** Quantification des chimiokines G-CSF, MCP-1, MIP-1α et MIP-1β attractantes et stimulant l'activation et la différenciation des cellules immunitaires, après 1 et 5 jours de challenge infectieux.
   **[****Fig. 10D****]** Quantification des lymphocytes T CD8+ et CD4+ infiltrés dans le tissu pulmonaire des souris BALB/c, après 1 et 5 jours de challenge infectieux.
   **[****Fig. 10E****]** Quantification des lymphocytes B infiltrés dans le tissu pulmonaire des souris BALB/c, après 1 et 5 jours de challenge infectieux.
   **[****Fig. 10F****]** Quantification des macrophages et neutrophiles infiltrés dans le tissu pulmonaire des souris BALB/c, après 1 et 5 jours de challenge infectieux.
   **[****Fig. 10G****]** Score cumulatif d'histopathologie pulmonaire, après 5 jours de challenge infectieux.

### DESCRIPTION DETAILLEE DE L'INVENTION

### Souches virales de Metapneumovirus humain (HMPV)

Les virus hMPV ont été identifiés en 2001 comme faisant partie de la famille des *Pneumoviridae.*

L'organisation génomique du hMPV est analogue au hRSV. Il est sous-divisé en plusieurs ORFs (*Open Reading Frame*) codant pour les protéines N, P, M, F, M2 (M2-1 et M2-2), SH, G et L. La structure génomique générale est représentée pour les virus rC-8543 et rCAN98-75 en figures 1A et 1B.

Les virus membres de la famille des *Pneumoviridae* expriment à leur surface trois glycoprotéines dénommées protéine F, G et SH.

La glycoprotéine F de fusion, très conservée entre les différents sous-groupes de hMPV, est impliquée dans la pénétration du virus dans les cellules cibles, et la formation de syncytium, c'est-à-dire de grandes cellules multi-nucléées issues de la fusion de cellules individuelles suite à leur infection par le virus. La glycoprotéine F est aussi considérée comme étant l'antigène majeur des virus hRSV et hMPV, permettant d'induire la production d'anticorps neutralisants.

La protéine G est une protéine membranaire de type II, son extrémité C-terminale étant à l'extérieur de la cellule. Cette protéine est non essentielle pour l'assemblage des constituants viraux, et pour la réplication *in vitro.* Pour le virus hRSV, il a été montré que la délétion du gène codant cette protéine atténuait la virulence de la souche lors de l'infection de tractus respiratoires de souris.

La protéine SH est également une protéine membranaire de type II, dont la fonction dans le cycle viral est encore inconnue. Dans le cas du virus hRSV, la délétion du gène codant pour la protéine SH génère un virus recombinant capable de se reproduire *in vitro,* et dont la virulence est atténuée dans le tractus respiratoire supérieur des souris, mais non dans la partie inférieure dudit tractus.

L'analyse génétique de souches cliniques du hMPV a permis de définir deux groupes majeurs (sérotypes A et B) et quatre sous-groupes « mineurs » (A1, A2, B1 et B2), basés principalement sur la variabilité de séquence des glycoprotéines de surface d'attachement (G) et de fusion (F). Il a ensuite été montré que ces groupes pouvaient encore être sous-divisés en sous-lignées telles que A2a et A2b (Huck *et al.,* 2006).

Parmi les souches virales largement étudiées, on peut citer la souche NL 00-1, appartenant au sérotype A1 ; la souche CAN 97-83 appartenant au sérotype A2 ; et la souche CAN 98-75, appartenant au sérotype B2.

La présente invention est basée sur une souche virale de *metapneumovirus* humain, dénommée rC-85473, isolée à partir d'un échantillon de patient au Canada, notamment référencée dans l'article (Hamelin *et al.,* 2010).

L'utilisation de la protéine F de cette souche, en combinaison avec la protéine M, a été proposée comme stratégie vaccinale (Aerts *et al.,* 2015a).

L'utilisation de cette souche virale rC-85473 inactivée par un traitement à la formaline, en tant que souche vaccinale vivante inactivée, a été décrite dans l'article (Palavecino *et al.,* 2015). Une version inactivée par la chaleur de la souche rC-85473 a également été testée (Hamelin *et al.,* 2007).

Plus récemment, les fortes capacités de fusion de cette souche ont été étudiées en détails, sur la base de la séquence protéique de sa protéine F. (Dubois *et al.,* 2017).

La séquence du gène F et du gène M de la souche rC-85473 sont référencés dans GenBank, respectivement aux numéros d'accès KM408076.1 et KM408077.1.

La séquence génomique complète de cette souche virale rC-85473, comprenant 13394 nucléotides, est ici divulguée pour la première fois : elle est représentée dans la liste des séquences en annexe, sous la référence SEQ ID NO. 1.

Le tableau 4 ci-dessous présente le pourcentage d'identité de cette souche rC-85473 avec trois autres souches virales de *metapneumovirus* humain : CAN 97-83, CAN 98-75 et NL 00-1.

**Tableau 4 - Pourcentage d'identité de séquence nucléotidique pour chaque ORF (l'identité de la séquence d'acides aminés est indiquée entre parenthèses) entre le virus rC-85473 et d'autres virus hMPV**

| **ORF** | **N** | **P** | **M** | **F** | **M2-1** | **M2-2** | **SH** | **G** | **L** |
|---|---|---|---|---|---|---|---|---|---|
| **CAN 97-83 (A2)** | 94 (99) | 91 (95) | 93 (99) | 94 (98) | 94 (96) | 94 (94) | 90 (85) | 81 (68) | 94 (98) |
| **CAN98-75 (B2)** | 86 (96) | 81 (85) | 85 (97) | 84 (94) | 85 (94) | 85 (88) | 70 (61) | 56 (39) | 84 (93) |
| **NL 00-1 (A1)** | 98 (99) | 97 (98) | 98 (99) | 97 (99) | 98 (98) | 98 (97) | 97 (96) | 93 (89) | 98 (99) |

Au regard de la forte homologie de séquence observée avec la souche NL 00-1, il peut être conclu que cette souche rC-85473 est membre du sous-groupe A1.

La souche rC-85473 se caractérise par de grandes capacités fusogènes, lui permettant de pénétrer dans les cellules cibles à une haute fréquence / un haut degré d'infection. Cette grande capacité fusogénique de cette souche permet de générer des syncytia particulièrement étendus. Les syncytia sont des cellules géantes multinucléées. Le virus rC-85473 a une forte capacité à induire la fusion cellulaire *in vitro,* conduisant à la formation de ces syncytia très étendus, constitués d'un très grand nombre de noyaux cellulaires.

Il a été montré que 5 acides aminés du domaine fonctionnel HRA de la protéine F, acides aminés uniques parmi les autres souches hMPV connues, sont en partie responsables de ce phénotype hyperfusogénique. (Aerts *et al.*, 2015b ; Dubois *et al.,* 2017).

Selon une mise en oeuvre préférée de la présente invention, la séquence peptidique de la protéine F de la souche virale atténuée dérivée de la souche rC-85473 comprend ces 5 acides aminés du domaine fonctionnel HRA tels que décrits dans (Dubois *et al.*, 2017).

Selon une mise en oeuvre préférée de la présente invention, la séquence peptidique de la protéine F de la souche virale atténuée dérivée de la souche rC-85473 n'est pas modifiée, et est donc identique à la séquence peptidique de la protéine F de la souche rC-85473 telle que décrite dans (Dubois *et al.*, 2017).

### Technologie de génétique inverse

La technologie de génétique inverse permet de créer, à partir d'une séquence d'acide nucléique, ADN ou ARN, et de cellules hôtes appropriées, un virus encapsidé fonctionnel.

La souche recombinante rC-85473 de *metapneumovirus* humain a ainsi été obtenue à partir d'une souche clinique désignée C-85473, isolée chez un patient.

Le premier système de génétique inverse appliqué au virus hMPV a été décrit en 2004 (Biachesi *et al.*, 2004). Cette technologie est également présentée dans l'article (Aerts *et al.*, 2015b).

Le principe de cette technologie, qui permet la production de virus hMPV recombinants, repose sur l'utilisation d'une lignée cellulaire de rein de hamster (BHK-21) modifiée pour exprimer constitutivement l'ARN polymérase du bactériophage T7 (cellules BHK-T7 ou BSR-T7/5).

Les éléments génomiques sont répartis dans cinq éléments plasmidiques : un plasmide codant l'antigénome du virus hMPV et 4 plasmides « satellite », codant pour les protéines virales de la machinerie de transcription (L, P, N et M2-1).

Après co-transfection du plasmide antigénomique hMPV et des quatre plasmides « satellite » dans ces cellules, un brin d'ARN correspondant au brin génomique viral (brin d'ARN négatif), est transcrit par la T7polymérase à partir de son promoteur.

Les quatre protéines impliquées dans la transcription virale du hMPV sont également exprimées par les cellules hôtes transfectées pour constituer un complexe RNA-dependant RNA polymerase (RdRP) actif. Cette polymérase virale fonctionnelle transcrit ainsi l'ARN génomique en ARNm viral puis le réplique en de nouvelles molécules d'ARN génomique viral, via la transcription de brins matrice.

La traduction et l'assemblage des protéines virales avec l'ARN génomique permettent ainsi le bourgeonnement de particules infectieuses hMPV à partir de la membrane cytoplasmique des cellules BHK-T7 transfectées. Ensuite, l'amplification des virus recombinants est permise grâce à l'ajout en co-culture de cellules LLC-MK2 (ATCC CCL-7), permissives à l'infection.

Ainsi, à partir d'une séquence génomique d'une souche virale particulière, il est possible de produire des virus recombinants grâce à ces cellules modifiées, accessibles dans le commerce.

### Souche virale atténuée dérivée de la souche rC-85473

La présente invention concerne une souche virale atténuée dérivée d'une souche de *metapneumovirus* humain comprenant la séquence génomique représentée par la séquence SEQ ID NO. 1, ladite souche atténuée comprenant une ou des modifications génétiques de ladite séquence SEQ ID NO. 1 atténuant la virulence de ladite souche, caractérisée en ce que les modifications de la séquence SEQ ID NO.1 consistent en l'inactivation du gène codant pour la protéine SH et/ou l'inactivation du gène codant pour la protéine G.

Dans la présente demande, les termes « un virus » et « une souche virale » sont utilisés indifféremment pour désigner une souche virale particulière, telle qu'identifiée précédemment. Au sens de l'invention, on entend par « souche dérivée » une souche virale recombinante obtenue par l'introduction de modifications génétiques dans le génome d'une souche virale dite « souche d'origine ». La souche d'origine est avantageusement une souche sauvage, par exemple un isolat clinique.

Les modifications génétiques introduites dans la souche d'origine ont toutes pour objet d'atténuer la virulence de ladite souche d'origine, et non de modifier l'identité de son génome. Ces modifications génétiques ne concernent que des gènes codant pour des protéines non essentielles pour la réplication du virus, autrement dites « protéines accessoires », telles que les protéines SH et G. Dans cette souche atténuée modifiée génétiquement, la séquence peptidique de la protéine F de la souche rC-85473 d'origine n'est pas modifiée, et présente donc la même séquence peptidique que la souche d'origine.

La virulence d'une souche virale correspond au degré de rapidité de multiplication d'un virus dans un organisme donné, donc à sa vitesse d'envahissement. Par « atténuer la virulence », on entend donc diminuer la vitesse d'envahissement d'un virus dans un organisme.

Cette atténuation peut prendre la forme d'une diminution des capacités de réplication de la souche virale, et/ou d'une diminution de sa capacité d'infection des cellules cibles, et/ou encore d'une diminution de la pathologie induite par l'infection virale de l'organisme.

Ainsi, par « souche virale atténuée » on entend, au sens de l'invention, un virus recombinant dont la virulence est diminuée par rapport à celle de la souche virale d'origine, c'est-à-dire inférieure à celle de la souche virale d'origine.

Pour mesurer la virulence d'une souche virale, des tests *in vitro*, *ex vivo* ou *in vivo* peuvent être réalisés, comme par exemple des tests de capacité réplicative *in vitro* (mesurée par titration TCID50/ml ou PCR quantitative), le suivi par observation microscopique de l'évolution des effets cytopathiques *in vitro* et ex *vivo,* ou le suivi des signes cliniques de la pathologie, l'observation histopathologique pulmonaire et la mesure de titres viraux pulmonaires dans un modèle d'infection *in vivo.*

Pour comparer les effets physiologiques d'une souche sauvage et d'une souche atténuée, il est également possible de mesurer / déterminer différents paramètres physiologiques modifiés au niveau du poumon et/ou du système immunitaire d'un organisme hôte du virus.

Comme cela est présenté dans les exemples 7, 8 et 9 de la présente demande, les effets viro-induits suivants peuvent être évalués pour comparer l'effet physiologique de souches virales atténuées, soit entre elles soit avec la souche virale sauvage :
- La sécrétion de cytokines pro-inflammatoires (par les cellules de l'épithélium et/ou par les macrophages);
- La sécrétion de chimiokines ;
- La sécrétion de facteurs de croissance ;
- Le taux d'inflammation pulmonaire (score calculé par évaluation de l'intensité de l'inflammation des tissus) ; et
- Le phénotypage des cellules immunitaires recrutées sur le site de l'infection *in vivo.*

Ces diverses mesures permettent de connaitre de manière plus précise l'effet physiologique de chaque souche virale, et de choisir en fonction de ces caractéristiques laquelle serait la plus adaptée pour développer un vaccin viral vivant atténué.

### Modifications génétiques introduites dans une souche virale pour obtenir son atténuation

La présente invention concerne une souche virale atténuée dérivée d'une souche de *metapneumovirus* humain comprenant la séquence génomique représentée par la séquence SEQ ID NO. 1, ladite souche atténuée comprenant une ou des modifications génétiques de ladite séquence SEQ ID NO. 1 atténuant la virulence de ladite souche, caractérisée en ce que les modifications de la séquence SEQ ID NO.1 consistent en l'inactivation du gène codant pour la protéine SH et/ou l'inactivation du gène codant pour la protéine G.

Par « modifications génétiques atténuant la virulence de ladite souche », on entend des modifications génétiques relatives à des gènes codant pour des protéines non essentielles à la réplication du virus, les protéines G et SH, présentées ci-dessus.

Autrement dit, la présente demande concerne une souche virale atténuée dérivée d'une souche de *metapneumovirus* humain comprenant la séquence génomique représentée par la séquence SEQ ID NO. 1, ladite souche atténuée ayant été modifiée génétiquement exclusivement pour atténuer sa virulence, caractérisée en ce que les modifications de la séquence SEQ ID NO.1 consistent en l'inactivation du gène codant pour la protéine SH et/ou l'inactivation du gène codant pour la protéine G.

Diverses modifications génétiques permettant d'atténuer la virulence d'une souche virale sont connues de l'Homme du métier. Ces modifications peuvent être introduites dans le génome de la souche d'origine rC-85473.

Les modifications génétiques désignent, au sens de l'invention, toutes les modifications d'une séquence nucléotidique originale telles que la délétion d'un ou plusieurs nucléotides, l'ajout d'un ou plusieurs nucléotides, et le remplacement d'un ou plusieurs nucléotides. Ces modifications comprennent notamment toutes les modifications permettant de décaler le cadre de lecture génétique, ou d'introduire un codon stop au milieu d'une séquence codante, ou la délétion de tout ou partie d'une ou plusieurs séquences codantes.

Parmi les modifications génétiques destinées à atténuer la virulence d'une souche virale, on connait notamment les modifications génétiques permettant d'inactiver voire de supprimer un ou plusieurs gènes codant pour des protéines non essentielles pour la réplication du virus en culture.

Des souches virales atténuées de metapneumovirus humains ont été obtenues par l'inactivation, en particulier par la délétion des gènes codant pour les protéines accessoires SH, G et M2-2.

Toutefois, en fonction des caractéristiques de la souche virale d'origine à partir de laquelle les souches atténuées sont dérivées, les caractéristiques fonctionnelles des souches virales atténuées pourront être très différentes, la virulence des souches dépendant à la fois du génome d'origine et des modifications génétiques apportées.

Selon un premier aspect de l'invention, la souche virale atténuée selon l'invention est caractérisée en ce que les modifications de la séquence SEQ ID NO.1 comprennent l'inactivation du gène codant pour la protéine SH.

Plus particulièrement, la souche virale atténuée selon l'invention est caractérisée en ce que la modification génétique de la séquence SEQ ID NO.1 atténuant la virulence de ladite souche consiste en une inactivation du gène codant pour la protéine SH.

Au sens de l'invention, l'inactivation d'un gène désigne le fait que ce gène est modifié de telle sorte que le produit du gène n'est plus exprimé, ou exprimé sous une forme non active, ou exprimé en une quantité si faible que l'activité de cette protéine est inexistante. Cette inactivation d'un gène peut être effectuée par toutes les techniques bien connues de l'Homme du métier.

En particulier, l'inactivation d'un gène peut être obtenue par l'introduction d'une mutation ponctuelle dans le gène, par la délétion partielle ou totale des séquences codantes du gène, ou encore par modification du promoteur du gène. Ces différentes modifications génétiques seront effectuées selon l'un quelconque des techniques de biologie moléculaire bien connues de l'Homme du métier.

Au sens de l'invention, on entend par « délétion d'un gène » le retrait du génome de la souche virale d'une partie significative de la séquence codante de ce gène, notamment :
- Lorsqu'il s'agit d'une délétion partielle, d'au moins 50%, 60%, 70%, 80%, 90% ou 95% de ladite séquence codante ;
- Lorsqu'il s'agit d'une délétion complète, de 100% de ladite séquence codante.

Selon un mode de réalisation préféré, dans la souche virale atténuée selon l'invention, le gène codant pour la protéine SH est totalement délété, c'est-à-dire que toute (100% de) la séquence codante pour la protéine SH a été retirée de la séquence d'origine SEQ ID NO. 1.

En particulier, ladite souche virale comprend la séquence nucléotidique telle que représentée en SEQ ID NO. 2. Plus spécifiquement, la séquence de ladite souche virale consiste en la séquence nucléotidique telle que représentée en SEQ ID NO. 2.

Comme cela est illustré dans les exemples, cette souche atténuée désignée ΔSH- rC-85473 présente les caractéristiques suivantes :
- *In vitro,* l'infection et la réplication sur des cellules en culture sont normales (pas de différence avec les caractéristiques de la souche d'origine);
- *In vivo,* l'infection de souris avec ce virus recombinant ΔSH-rC-85473 n'induit que très peu de perte de poids, à la différence d'une infection avec la souche originale ; la survie des souris infectées est de 100%, alors que ce virus atténué se réplique dans les tissus pulmonaires ;
- *In vivo,* une immunisation préalable de souris avec cette souche atténuée protège à 100% les souris challengées par une dose létale de virus rC-85473 sauvage. Cette protection est également illustrée par l'absence de détection de virus infectieux rC-85473 dans les poumons 5 jours après le challenge ; et par un titre élevé de microneutralisation contre la souche virale homologue (C-85473, de sérotype A) et contre au moins une souche hétérologue (CAN98-75, de sérotype B) des sérums à J+21 après le challenge infectieux ;
- *In vitro,* la capacité d'attachement aux cellules LLC-MK2 en culture et d'infection cellulaire est bien meilleure pour le virus atténué ΔSH- rC-85473 (environ 60% de cellules infectées) en comparaison de celles du virus ΔSH-rCAN98-75 (environ 40% de cellules infectées) ;
- *Ex vivo,* l'infection d'un modèle d'épithélium respiratoire par cette souche atténuée induit la sécrétion de cytokines et chimiokines dans une quantité équivalente ou inférieure à celle induite par la souche sauvage ; la réponse inflammatoire est toutefois réduite par rapport à celle induite par la souche sauvage (figures 7A, 7B);
- *In vitro,* l'infection de macrophages par cette souche atténuée induit la sécrétion de cytokines pro-inflammatoires, mais dans une cinétique différente de celle viro-induite par la souche sauvage (figures 8B, 8C);
- *In vivo,* l'inflammation viro-induite des tissus pulmonaires est significativement plus faible suite à l'infection par cette souche atténuée, qu'avec la souche sauvage (figure 9A) ; toutefois, le recrutement des cellules immunitaires est toujours assuré (figure 9B).

Selon un deuxième aspect de l'invention, la souche virale atténuée selon l'invention est caractérisée en ce que les modifications de la séquence SEQ ID NO.1 comprennent l'inactivation du gène codant pour la protéine G.

Plus particulièrement, la souche virale atténuée selon l'invention est caractérisée en ce que la modification génétique de la séquence SEQ ID NO.1 atténuant la virulence de ladite souche consiste en une inactivation du gène codant pour la protéine G.

Selon un mode de réalisation préféré, dans la souche virale atténuée selon l'invention, le gène codant pour la protéine G est totalement délété, c'est-à-dire que toute (100% de) la séquence codante pour la protéine G a été retirée de la séquence d'origine SEQ ID NO. 1.

En particulier, ladite souche virale comprend la séquence nucléotidique telle que représentée en SEQ ID NO. 3. Plus spécifiquement, la séquence de ladite souche virale consiste en la séquence nucléotidique telle que représentée en SEQ ID NO. 3.

Comme cela est illustré dans les exemples, cette souche atténuée désignée ΔG- rC-85473 présente les caractéristiques suivantes :
- *In vitro,* dans le modèle cellulaire LLC-MK2, l'infection et la réplication sur des cellules en culture sont normales (pas de différence avec les caractéristiques de la souche d'origine);
- *In vivo,* l'infection de souris infectées avec ce virus recombinant ΔG-rC-85473 n'induit que très peu de perte de poids ; la survie des souris infectées est de 100% ; alors que ce virus se réplique dans les tissus pulmonaires ;
- *In vivo,* une immunisation préalable de souris avec cette souche atténuée protège à 100% les souris challengées par une dose létale de virus rC-85473 sauvage. Cette protection est également illustrée par l'absence de détection de virus infectieux et de génome rC-85473 sauvage dans les poumons à J+5 après challenge ; et par un titre élevé de microneutralisation contre la souche virale homologue (C-85473, de sérotype A) et contre au moins une souche hétérologue (CAN98-75, de sérotype B) des sérums à J+21 après challenge infectieux ;
- *In vitro,* la capacité d'attachement aux cellules LLC-MK2 en culture et d'infection cellulaire est bien meilleure pour le virus atténué ΔG- rC-85473 (environ 60% de cellules infectées) en comparaison de celles du virus ΔG-rCAN98-75 (moins de 20% de cellules infectées).
- *Ex vivo,* l'infection d'un modèle d'épithélium respiratoire par cette souche atténuée induit la sécrétion de cytokines et chimiokines dans une quantité nettement inférieure à celle induite pas la souche sauvage (figure 7);
- *In vitro,* l'infection de macrophages par cette souche atténuée induit la sécrétion de TNF-α, de manière équivalente à la sécrétion viro-induite par la souche sauvage ; mais n'induit que très faiblement la sécrétion d'IL-1β (Figures 8B, 8C) ;
- *In vivo,* l'inflammation viro-induite des tissus pulmonaires est significativement plus faible suite à l'infection par cette souche atténuée, qu'avec la souche sauvage ou qu'avec la souche atténuée ΔSH- rC-85473 (figure 9A).

Selon un troisième aspect de l'invention, la souche virale atténuée selon l'invention est caractérisée en ce que les modifications de la séquence SEQ ID NO.1 comprennent en l'inactivation des deux gènes codant pour la protéine G et la protéine SH.

Plus particulièrement, la souche virale atténuée selon l'invention est caractérisée en ce que les modifications génétiques de la séquence SEQ ID NO.1 atténuant la virulence de ladite souche consistent en une inactivation des deux gènes codant l'un pour la protéine SH et l'autre pour la protéine G.

Selon une mise en oeuvre particulière, l'inactivation des deux gènes correspond à la délétion complète de l'un ou de l'autre ou des deux gènes codant pour les protéines G et SH.

De plus, il est entendu que toute autre modification génétique permettant d'atténuer la virulence de la souche rC-85437 pourra être introduite dans le génome de ladite souche, représenté par la séquence SEQ ID NO.1.

### Introduction de gènes exogènes au sein du génome de la souche virale rC-85473

Selon un aspect de l'invention, la séquence nucléotidique de la souche virale atténuée selon l'invention pourra être modifiée génétiquement par l'introduction d'au moins un gène exogène.

Ainsi, la souche virale atténuée selon l'invention présente une séquence génomique qui comprend au moins un gène exogène. Ce gène exogène pourra en particulier être un gène codant pour un antigène viral.

Au sens de l'invention, on entend par « antigène viral » un élément protéique ou d'une autre nature, exprimé par un virus, que le système immunologique d'un individu reconnaît comme étranger et qui provoque une réponse chez ledit individu par la production d'anticorps spécifiques et/ou la stimulation d'une réponse immunitaire cellulaire.

Les antigènes viraux pourront en particulier être sélectionnés parmi les antigènes exprimés par au moins un virus influenza, ou par au moins un virus de la famille des *Pneumoviridae,* tel que le virus hRSV, ou par au moins un virus de la famille des *Paramyxoviridae*, tel que le virus parainfluenza.

Plus particulièrement, ledit antigène viral pourra être choisi parmi tout ou partie de la protéine F du virus hRSV, et tout ou partie de l'hémagglutinine des virus influenza ou parainfluenza.

Cette souche virale atténuée permettra, lors de son administration à un patient, de générer une réponse immunitaire multiple, à la fois contre l'antigène viral exprimé et contre le virus hMPV.

Une telle souche permettant d'obtenir une réponse immunitaire combinée contre plusieurs virus, à la suite d'une seule administration, est très avantageuse.

De plus, une telle souche virale atténuée comprenant au moins un gène exogène pourra être utilisée *in vivo, ex vivo* ou *in vitro,* en tant que vecteur d'expression d'au moins un gène exogène dans des cellules cibles du *metapneumovirus* humain.

On entend par 'cellules cibles du *metapneumovirus* humain' les cellules épithéliales du tractus respiratoire des individus susceptibles d'être infectés par ce virus. Cette expression désigne également toutes les lignées cellulaires permettant la réplication *in vitro* dudit virus.

### Souche virale atténuée pour son utilisation en tant que médicament

La présente invention concerne également une souche virale atténuée telle que définie ci-dessus, pour son utilisation en tant que médicament.

En effet, cette souche peut être utilisée, notamment, pour traiter ou prévenir des infections virales.

Le terme « traiter » désigne le fait de combattre l'infection par un virus dans un organisme humain ou animal. Grâce à l'administration d'au moins une composition selon l'invention, le taux d'infection virale (titre infectieux) dans l'organisme va diminuer, et de préférence le virus va disparaitre complètement de l'organisme. Le terme « traiter » désigne aussi le fait d'atténuer les symptômes associés à l'infection virale (syndrome respiratoire, défaillance rénale, fièvre, etc...).

Au sens de l'invention, le terme « prévenir » désigne le fait d'empêcher, ou du moins de diminuer le risque d'apparition, d'une infection dans un organisme humain ou animal. Grâce à l'administration d'au moins une souche virale atténuée selon l'invention, les cellules humaines ou animales dudit organisme deviennent moins permissives à l'infection, et sont ainsi plus à même de ne pas être infectées par ledit virus.

Plus spécifiquement, l'invention concerne une souche virale atténuée telle que définie ci-dessus, pour son utilisation pour prévenir et/ou traiter les infections par des virus de la famille des *Pneumoviridae.*

Il est entendu que cette souche virale atténuée sera de préférence intégrée à une composition vaccinale comprenant un véhicule pharmaceutiquement acceptable, adapté pour la mise en suspension de ladite souche et pour son administration.

Ladite composition vaccinale comprend au moins une souche virale atténuée selon l'invention, permettant de stimuler de manière spécifique le système immunitaire d'un organisme humain ou animal.

Ainsi, cette composition vaccinale comprend au moins une souche virale vivante atténuée qui joue le rôle d'antigène, c'est à dire de composé induisant une réponse immunitaire spécifique dans l'organisme, qui en conservera la mémoire.

Une telle composition vaccinale pourra être utilisée en tant que vaccin préventif, c'est-à-dire destiné à stimuler une réponse immunitaire spécifique avant l'infection d'un organisme par un virus pathogène.

Une telle composition vaccinale pourra également être utilisée en tant que vaccin thérapeutique, c'est-à-dire destiné à stimuler une réponse immunitaire spécifique de manière concomitante avec l'infection d'un organisme par ledit virus pathogène.

La présente invention concerne également une méthode pour prévenir et/ou traiter les infections par des *Pneumoviridae* chez l'Homme, comprenant l'administration, à des individus susceptibles d'être infectés par de tels virus, d'au moins une souche virale atténuée telle que décrite précédemment.

La présente invention concerne également une méthode pour prévenir et/ou traiter les infections par des *Pneumoviridae* chez l'Homme, comprenant l'administration, à des individus susceptibles d'être infectés par de tels virus, d'au moins une composition vaccinale comprenant au moins une souche virale atténuée telle que décrite précédemment.

Selon une mise en oeuvre spécifique de l'invention, les infections sont des infections par des *metapneumovirus* humains.

Selon une autre mise en oeuvre spécifique de l'invention, les infections sont des infections par des *orthopneumovirus,* tel que le virus respiratoire syncytial humain (hRSV).

L'invention concerne également une souche virale atténuée dont la séquence comprend au moins un gène exogène codant pour un antigène viral, pour son utilisation pour prévenir et/ou traiter les infections par des virus dont au moins un antigène viral est exprimé par ladite souche virale atténuée.

Selon une mise en oeuvre particulière, une souche virale atténuée selon l'invention comprenant, en tant qu'antigène viral exogène, la protéine F d'un virus hRSV, pourra être utilisée pour prévenir et/ou traiter les infections par un virus hRSV.

Selon une autre mise en oeuvre particulière, une souche virale atténuée selon l'invention comprenant, en tant qu'antigène viral exogène, l'hémagglutinine d'un virus influenza, pourra être utilisée pour prévenir et/ou traiter les infections par un virus influenza.

### Composition pharmaceutique comprenant au moins une souche virale atténuée

La présente invention concerne également une composition vaccinale comprenant, dans un véhicule pharmaceutiquement acceptable, au moins une souche virale atténuée selon l'invention, et optionnellement un adjuvant.

Selon l'invention, le terme « véhicule pharmaceutiquement acceptable » désigne des véhicules ou des excipients, c'est à dire des composés ne présentant pas d'action propre sur l'infection ici considérée. Ces véhicules ou excipients sont pharmaceutiquement acceptables, ce qui signifie qu'ils peuvent être administrés à un individu ou à un animal sans risque d'effets délétères significatifs ou d'effets indésirables rédhibitoires.

Il est entendu que la composition vaccinale selon l'invention comprend au moins une quantité efficace de la souche virale atténuée. Par « quantité efficace », on entend au sens de l'invention une quantité de souche virale atténuée suffisante pour déclencher une réaction immunitaire dans l'organisme auquel elle est administrée.

Les compositions vaccinales l'utilisation selon la présente invention sont adaptées pour une administration orale, sublinguale, par inhalation, sous-cutanée, intramusculaire ou intraveineuse.

Selon une mise en oeuvre particulière de l'invention, la composition vaccinale selon l'invention est caractérisée en ce qu'elle est sous une forme galénique destinée à une administration par inhalation.

L'inhalation désigne l'absorption par les voies respiratoires. C'est en particulier une méthode d'absorption de composés à des fins thérapeutiques, de certaines substances sous forme de gaz, de micro-gouttelettes ou de poudre en suspension.

L'administration de compositions pharmaceutiques ou vétérinaires par inhalation, c'est-à-dire par les voies nasale et/ou buccale, est bien connue de l'Homme du métier.

On distingue deux types d'administration par inhalation :
- l'administration par insufflation lorsque les compositions sont sous la forme de poudres, et
- l'administration par nébulisation lorsque les compositions sont sous la forme d'aérosols (suspensions) ou sous la forme de solutions, par exemple de solutions aqueuses, mises sous pression. L'utilisation d'un nébuliseur ou d'un pulvérisateur sera alors recommandée pour administrer la composition pharmaceutique ou vétérinaire.

La forme galénique considérée ici est donc avantageusement choisie parmi : une poudre, une suspension aqueuse de gouttelettes ou une solution sous pression.

La population d'individus à vacciner est principalement une population pédiatrique, c'est-à-dire constituée d'individus âgés de moins de 18 ans, et plus spécifiquement de jeunes enfants âgés de moins de 5 ans, et de nourrissons. En effet, les virus de la famille des *Pneumoviridae* infectent principalement ces individus, qui ont tendance à présenter une immunité moins forte que des individus plus âgés.

L'invention concerne également une composition vaccinale telle que présentée ci-dessus, pour son utilisation pour prévenir et/ou traiter les infections par des virus de la famille des *Pneumoviridae.*

### EXEMPLES

### Exemple 1 : Virus recombinants hMPV générés à partir des souches cliniques C-85473 et CAN98-75. 1A

Les constructions génétiques représentées en figures 1A et 1B ont été préparées afin que les virus soient détectables par expression de GFP (Green *Fluorescent Protein*), et pour introduire dans les souches de référence recombinantes rC-85473 et rCAN98-75 les modifications génétiques destinées à atténuer leur virulence :
- La délétion du gène codant pour la protéine SH (ΔSH) ;
- La délétion du gène codant pour la protéine G (ΔG).

Les séquences complètes de ces constructions génétiques sont présentées en SEQ ID NO. 4 (GFP rC-85473), SEQ ID NO.5 (GFP ΔSH-rC-85473), SEQ ID NO.6 (GFP ΔG-rC-85473) et SEQ ID NO. 8 (GFP rCAN98-75). Les séquences des souches virales dérivées de rCAN98-75 et couplées à la GFP ne sont pas représentées mais sont accessibles à l'Homme du métier, sur la base des autres constructions génétiques présentées.

Comme le montrent les photos des cellules LLC-MK2 infectées à une multiplicité d'infection (MOI) de 0,01, les virus étant visibles grâce à la GFP, les virus recombinants générés suivants : ΔSH-rCAN98-75, ΔG-rCAN98-75, ΔSH-rC-85473 et ΔG-rC-85473 sont fonctionnels et réplicatifs. Les cellules sont observées en microscopie à fluorescence à trois jours post-infection.

Les photos suggèrent que les virus recombinants rCAN98-75 semblent moins infectieux et moins réplicatifs que les virus recombinants rC-85473 : l'intensité de la fluorescence est plus faible, et les syncytia sont de dimensions plus réduites.

### Exemple 2 : Capacités réplicatives in vitro des virus recombinants rC-85473 et rCAN98-75.

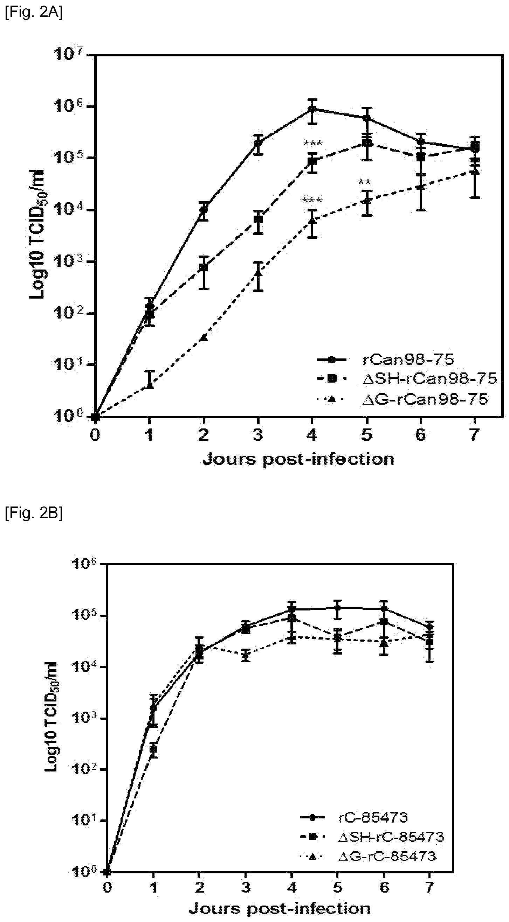

Des cellules LLC-MK2 ont été infectées séparément, à une multiplicité d'infection de 0.01, par les virus recombinants suivants :
- rCAN98-75, ΔSH-rCAN98-75 et ΔG-rCAN98-75 (figure 2A)
- rC-85473, ΔSH- rC-85473 et ΔG- rC-85473 (figure 2B)

Les surnageants cellulaires ont été récoltés chaque jour pendant 7 jours, en triplicat, puis leurs titres viraux ont été évalués en TCID50/ml. Les tests statistiques Two-way ANOVA ont été réalisés pour comparer chaque virus recombinant délété (ΔSH et ΔG) au virus recombinant sauvage.

Les figures 2A et 2B présentent les résultats obtenus : les capacités réplicatives et de production sont différentes en fonction de la nature de la souche d'origine, CAN98-75 ou C-85473, pour les virus recombinants atténués.

Les virus recombinants rC-85473, ΔSH- rC-85473 et ΔG- rC-85473 présentent des capacités réplicatives et de production bien meilleures que celles des virus rCAN98-75, ΔSH-rCAN98-75 et ΔG-rCAN98-75. Les virus ΔSH-rCAN98-75 et ΔG-rCAN98-75 sont les moins performants, alors que les virus ΔSH- rC-85473 et ΔG- rC-85473 présentent les meilleures capacités de production en culture sur des cellules LLC-MK2.

Dans les tableaux ci-dessous, sont représentés les titres moyens des stocks viraux produits et concentrés pour chaque virus recombinant rCAN98-75, ΔSH-rCAN98-75 et ΔG-rCAN98-75 (tableau 5) et rC-85473, ΔSH- rC-85473 et ΔG- rC-85473 (tableau 6).

Les titres viraux sont comptabilisés dans l'unité « TCID50 » qui représente la dernière dilution virale à laquelle 50% du tissu cellulaire est détruit par l'infection, ou montre des effets cytopathiques visibles (50% Tissue culture Infective Dose).

**Tableau 5.**

| **Virus recombinants** | **Titre moyen du stock concentré (TCID50/ml)** |
|---|---|
| rCAN98-75 | 4,37 E+07 |
| ΔSH -rCAN98-75 | 2,77 E+07 |
| ΔG -rCAN98-75 | 8,65 E+06 |

**Tableau 6.**

| **Virus recombinants** | **Titre moyen du stock concentré (TCID50/ml)** |
|---|---|
| rC-85473 | 3,92 E+07 |
| ΔSH -rC-85473 | 1,81 E+08 |
| ΔG -rC-85473 | 2,04 E+08 |

Ces titres viraux confirment les résultats précédents, à savoir que les souches virales atténuées dérivées de C-85437 présentent des capacités de réplication satisfaisantes et non diminuées par rapport à celles de la souche originale.

### Exemple 3 : Caractérisation in vivo des virus hMPV recombinants rCAN98-75 et rC-85473 sur un modèle d'infection virale de souris BALB/c

Des souris BALB/c ont été infectées par instillation intranasale avec :
- du milieu de culture OptiMem (mock) ou
- 2.5×10⁵ Fluorescent Focal Unit (FFU) des virus recombinants exprimant la GFP : rCAN98-75, ΔSH-rCAN98-75 et ΔG-rCAN98-75, ou
- 5×10⁵ TCID50 des virus n'exprimant pas la GFP : rC-85473, ΔSH-rC-85473 et ΔG-rC-85473.

Les deux doses de virus utilisées sont équivalentes (les virus sauvages produisent des effets équivalents chez les souris après infection).

Un suivi quotidien de poids et de survie des souris a été effectué pendant 14 jours (Moyenne de poids sur 10 souris ± SEM).

De plus, 5 jours après l'infection, 5 souris par groupe ont été euthanasiées pour une mesure de titres viraux infectieux pulmonaires en TCID50.

Des tests statistiques ont été réalisés (two-way ANOVA) pour comparer chaque virus à la condition mock. *p < 0.05, ** p < 0.01 and ***, p < 0.001.

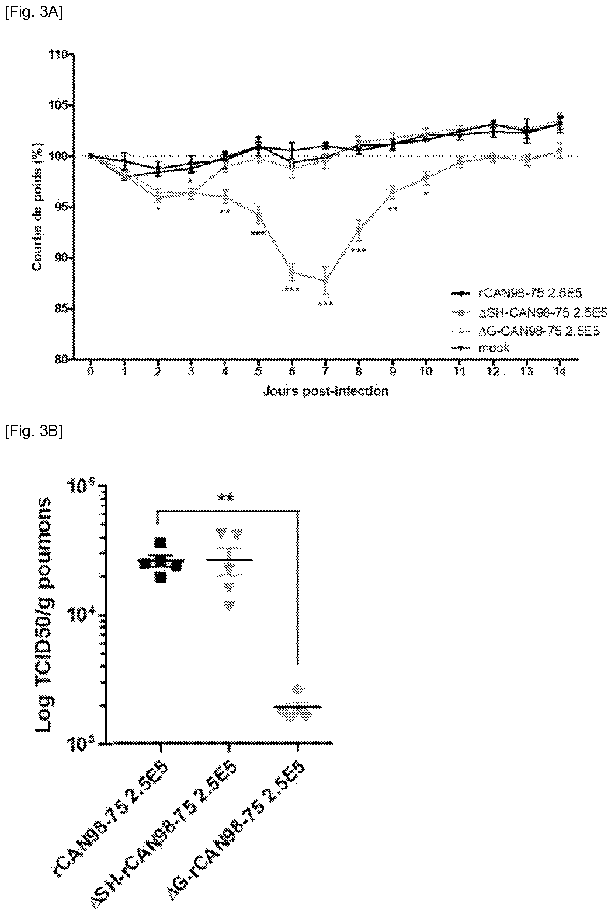

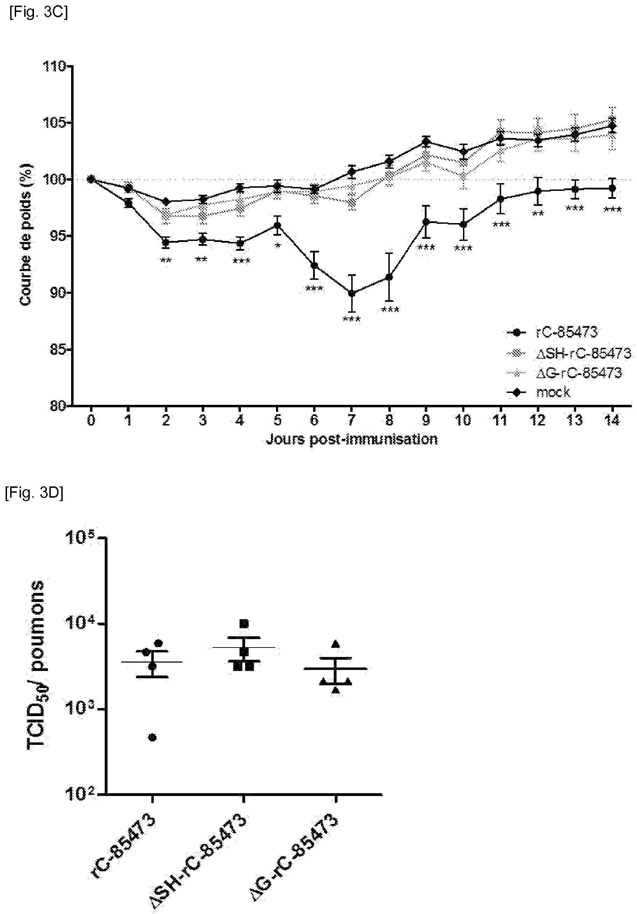

Les figures 3A et 3B montrent les résultats obtenus avec les virus rCAN98-75, ΔSH-rCAN98-75 et ΔG-rCAN98-75 ; les figures 3C et 3D les résultats obtenus avec les virus rC-85473, ΔSH-rC-85473 et ΔG-rC-85473.

Ces résultats indiquent que les virus recombinants ΔSH-rC-85473 ou ΔG-rC-85473 n'induisent que très peu de perte de poids ; en effet ces groupes de souris se comportent comme le groupe de souris non infectée.

Par ailleurs, les titres viraux pulmonaires (Fig. 3D) sont similaires à ceux observés dans le groupe infecté par le virus rC-85473, indiquant une réplication pulmonaire des virus atténués délétés.

Les résultats sont différents avec les virus recombinants ΔSH-rCAN98-75 et ΔG-rCAN98-75 : le virus ΔSH-rCAN98-75 induit une très forte perte de poids (Fig. 3A); alors que le virus ΔG-rCAN98-75 n'induit qu'une très faible diminution de poids (Fig. 3A), du fait de sa faible capacité à se répliquer dans les poumons (Fig. 3B).

### Exemple 4 : Propriétés protectrices in vivo des virus hMPV recombinants ΔG-rC-85473 et ΔSH-rC-85473 en modèle murin BALB/c suite à un challenge infectieux par le virus sauvage rC-85473

Le protocole d'immunisation et du challenge infectieux est présenté en figure 4A.

Des souris BALB/c ont été immunisées par instillation intranasale avec du milieu de culture OptiMem (témoin négatif, « mock ») ou 5×10⁵ TCID⁵⁰ des virus recombinants rC-85473, ΔSH-rC-85473 ou ΔG-rC-85473.

21 jours après l'immunisation, les souris ont été infectées (challenge infectieux) par instillation intranasale d'une dose létale (1×10⁶ TCID⁵⁰) du virus rC-85473 sauvage.

Un suivi quotidien de poids et de survie des souris a été effectué pendant 14 jours (Moyenne de poids sur 10 souris ± SEM, Figure 4B).

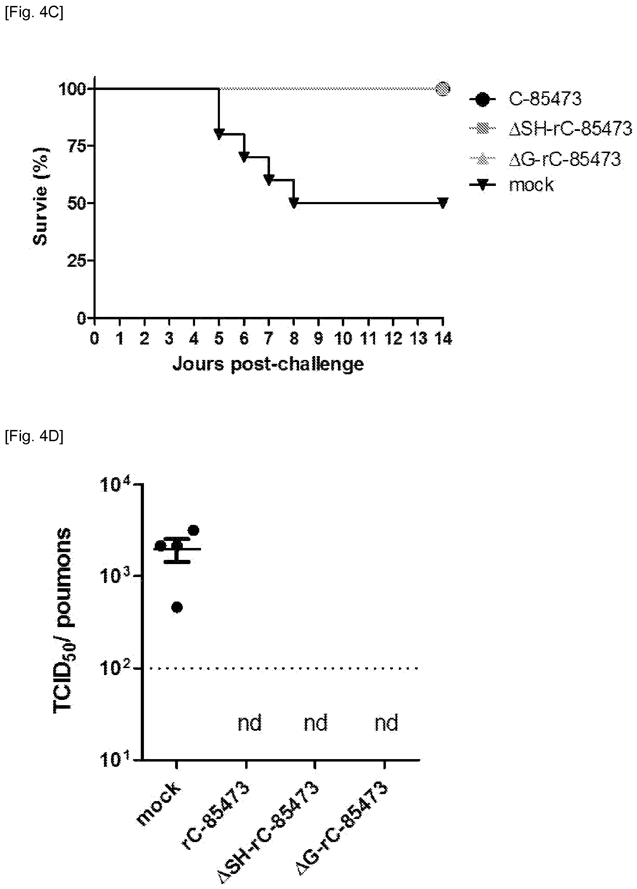

Un suivi quotidien de survie des souris a été effectué pendant 14 jours post-challenge, pour les souris des différents groupes (mock avec 50% de survie et immunisées par les virus recombinants rC-85473, ΔSH-rC-85473 ou ΔG-rC-85473, présentant 100% de survie). Les résultats sont présentés en Figure 4C.

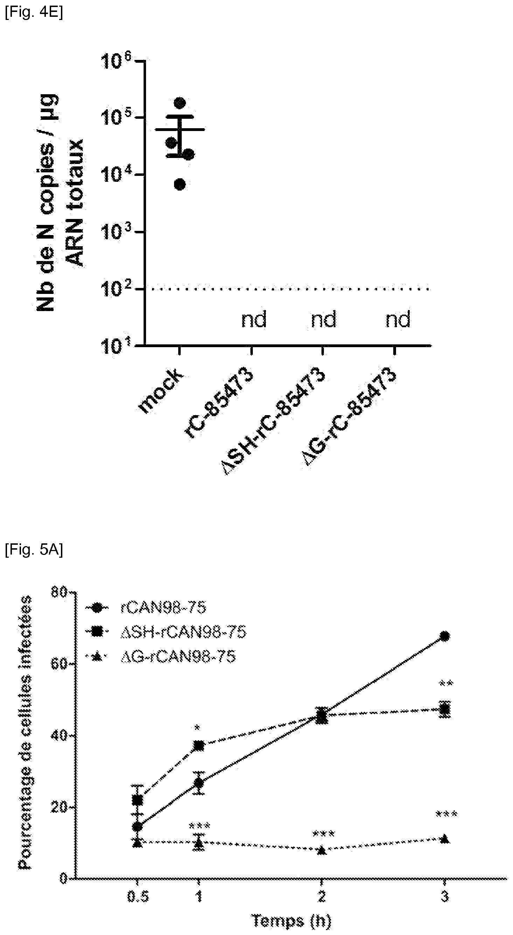

5 jours après le challenge infectieux (soit au jour 26 post-immunisation), 4 souris de chaque groupe ont été euthanasiées pour mesurer leurs titres viraux infectieux pulmonaires en TCID50 (Figure 4D) et en RTqPCR (gène N, Figure 4E).

Tests statistiques Two-way ANOVA pour comparer chaque virus à la condition mock. *p < 0.05, ** p < 0.01 and ***, p < 0.001.

Ces résultats confirment que les virus recombinants ΔSH-rC-85473 ou ΔG-rC-85473 n'induisent que très peu de perte de poids (les groupes de souris se comportent comme le groupe de souris non infectée) et que dans ces conditions expérimentales, protègent à 100% les souris challengées par une dose létale de virus rC-85473 sauvage. Cette protection est également illustrée par l'absence de détection de virus infectieux et de génome rC-85473 sauvage (nd) dans les poumons à J+5 après challenge et un titre élevé de microneutralisation des sérums à J+21 après challenge (> 160) des souris immunisées préalablement par les virus recombinants ΔSH-rC-85473 et ΔG-rC-85473.

Le tableau 7 ci-dessous présente les résultats d'essais de micro-neutralisation, réalisés à partir des sérums prélevés chez les souris des différents groupes à J+20 (1 jour avant le challenge infectieux) et à J+42 (21 jours après le challenge infectieux).

Après inactivation 30 minutes à 56°C et dilution des sérums, ceux-ci ont été incubés avec 100 TCID50 (c'est-à-dire 100 unités infectieuses telles que déterminées d'après le TCDI50/ml) de virus rC-85473 ou de virus rCAN98-75 pendant 2 heures à 37°C. Ce mix a ensuite été appliqué à des cellules LLC-MK2 pour une détermination de titre de neutralisation.

Le titre d'anticorps neutralisant est déterminé par la plus forte dilution de sérum à laquelle les cellules LLC-MK2, incubées avec le mix sérum+virus, ne montrent pas d'effets cytopathiques, c'est-à-dire sont considérées négatives pour l'infection (sur la base de tests effectués en duplicat).

**Tableau 7. Essai de microneutralisation**

| **Sérums** | **Jour 20 avant challenge infectieux** | **Jour 42, 21 jours après le challenge infectieux (souche C-85473)** | **Jour 42, 21 jours après le challenge infectieux (souche CAN98-75, sérotype B)** |
|---|---|---|---|
| Mock | ≤ 5 | 10 | 10 |
| rC-85473 | 5 | ≥ 160 | 80 |
| ΔSH rC-85473 | 5-10 | ≥ 160 | ≥ 160 |
| ΔG rC-85473 | 5-10 | ≥ 160 | 80 |

Les chiffres représentent la dilution des sérums testés à laquelle les titres d'anticorps spécifiques neutralisent efficacement l'infection virale (titre de microneutralisation).

Un résultat de 5 ou 10 est représentatif d'une primo réponse immunitaire (première rencontre avec le virus). Un résultat supérieur ou égal à 160 est caractéristique d'une réponse immunitaire maximale, et signifie que l'organisme avait déjà rencontré le virus précédemment et donc était immunisé.

Les souches virales dérivées de la souche d'origine rC-85473 permettent d'obtenir une réponse humorale neutralisante satisfaisante contre le virus hMPV C-85473 (sérotype A) et au moins contre le virus CAN98-75 (sérotype B), après immunisation préalable par les souches atténuées.

### Exemple 5 : Capacités d'attachement cellulaire in vitro des virus recombinants rC-85473 et rCAN98-75.

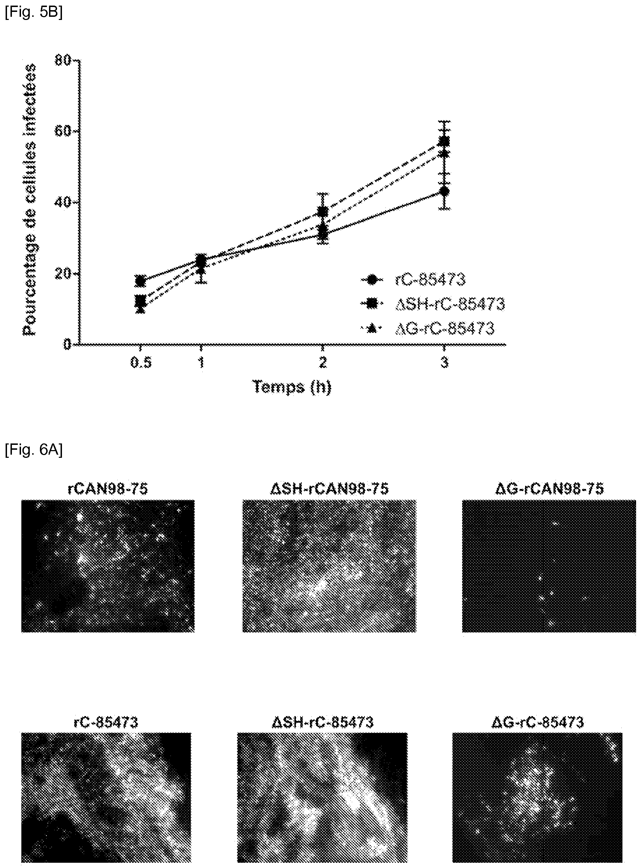

Des cellules LLC-MK2 ont été infectées (t=0) avec :
- le virus rCAN98-75 et ses formes atténuées ΔSH-rCAN98-75 et ΔG-rCAN98-75 (figure 5A) ;
- le virus rC-85473 et ses formes atténuées ΔSH-rC-85473 et ΔG-rC-85473 (figure 5B) ;

Les cellules ont été infectées sur glace, pendant 0.5, 1, 2 ou 3h pour faire varier le temps d'attachement des virus aux cellules, avant d'être lavées puis incubées à 37°C pendant 24h.

Le pourcentage de cellules infectées mesuré à 24h post-infection est représentatif de la quantité de virus capable de se lier aux cellules dans le temps imparti (0.5, 1, 2 ou 3h).

Les résultats indiquent des capacités d'attachement aux cellules LLC-MK2 en culture (et donc des capacités d'infection) différentes pour les virus recombinants en fonction de l'origine de leur génome CAN98-75 ou C-85473.

Les résultats montrent une capacité d'attachement cellulaire et d'infection cellulaire bien meilleure pour les virus atténués ΔSH- rC-85473 et ΔG-rC-85473 (environ 60% de cellules infectées) en comparaison de celles des virus ΔSH-rCAN98-75 (40%) et ΔG-rCAN98-75 (moins de 20% de cellules infectées).

### Exemple 6: Capacités réplicatives ex vivo des virus recombinants rC-85473 et rCAN98-75 en modèle 3D d'épithélium respiratoire humain reconstitué et cultivé à l'interface air-liquide.

Des épithéliums respiratoires humains reconstitués (MucilAir^{®} HAE, Epithelix) et maintenus en culture à l'interface air-liquide selon les instructions du fournisseur Epithelix, ont été utilisés pour réaliser ces expériences ex *vivo.*

Les photos présentées en figure 6A indiquent que les virus recombinants rCAN98-75 apparaissent moins infectieux et moins réplicatifs que les virus recombinants rC-85473 (plus faible intensité de la fluorescence et dimensions plus réduites des foyers d'infection).

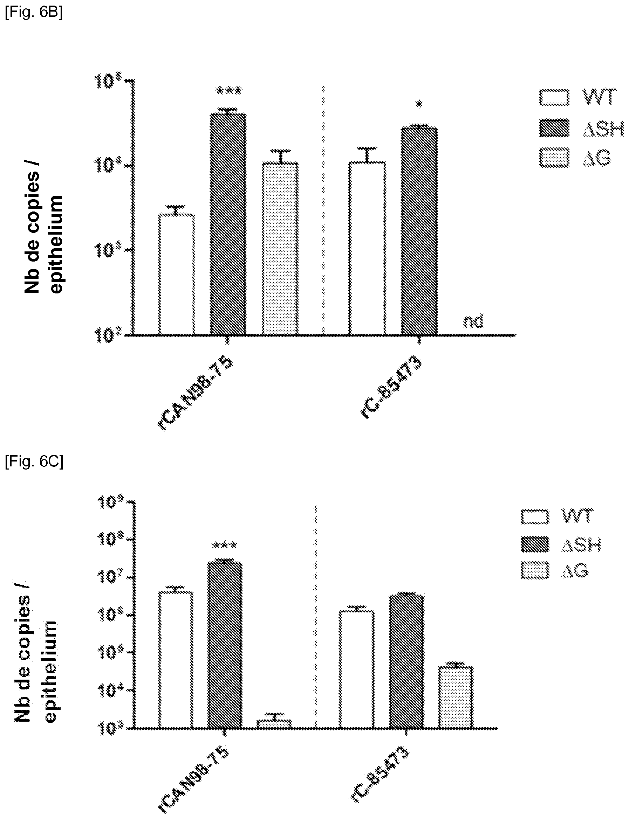

Les résultats de quantification de génome viral dans les épithéliums (figure 6C) confirment les capacités réplicatives des virus recombinants rC-85473, ΔSH- rC-85473 et ΔG- rC-85473, ainsi que des virus recombinants rCAN98-75 et ΔSH-rCAN98-75, à la différence du virus ΔG-rCAN98-75 très faiblement réplicatif.

Les résultats de quantification de génome viral à la surface du pôle apical des épithéliums (figure 6B) indiquent une production de virus recombinants rCAN98-75, ΔSH-rCAN98-75, ΔG-rCAN98-75, rC-85473 et ΔSH- rC-85473.

En revanche, aucun génome de virus ΔG- rC-85473 n'est détecté, indiquant l'absence de production de progéniture virale. Une hypothèse est que le virus ΔG-rC-85473 aurait perdu la capacité de bourgeonner à la surface des cellules infectées dans ce système ex *vivo* complexe et pluristratifié.

Ces différences seraient de nature à argumenter davantage en faveur du caractère inventif de notre invention, se focalisant sur les propriétés différentes entre virus recombinants de différentes origines en termes de souche virale hMPV, pour la protection de nos candidats vaccins vivants atténués ΔSH- rC-85473 et ΔG- rC-85473.

### Exemple 7 : Sécrétion de cytokines et chimiokines induites par l'infection des virus recombinants rC-85473 sur un modèle 3D d'épithélium respiratoire humain reconstitué et cultivé à l'interface air-liquide.

Des épithéliums respiratoires humains reconstitués (MucilAir^{®} HAE, Epithelix) maintenus en culture à l'interface air-liquide, selon les instructions du fournisseur Epithelix, ont été utilisés pour réaliser ces expériences *ex vivo.*

La quantification d'une sélection de cytokines et chimiokines sécrétées dans le milieu basal des épithéliums infectés témoigne de l'induction d'une réponse cellulaire différente en fonction des virus recombinants rC-85473 évalués (figure 7).
a) Le virus ΔG-rC-85473 induit la sécrétion d'une quantité significativement plus faible des différentes cytokines et chimiokines testées, en comparaison avec la souche sauvage rC-85473, à l'exception de la cytokine pro-inflammatoire TNF-α. Ces résultats sont concordants avec ceux présentés en figure 6, qui montrent que l'infectivité de ce virus en épithélium respiratoire humain reconstitué est significativement réduite par rapport à l'infectivité de la souche sauvage.
b) Le virus ΔSH-rC-85473, bien que sa capacité d'infection et de réplication en modèle d'épithélium respiratoire humain reconstitué soit augmentée par rapport à celle du virus recombinant sauvage rC-85473 (figure 6), induit la sécrétion d'une quantité équivalente ou inférieure du panel de cytokines et chimiokines évaluées par rapport à celle viro-induite par le virus sauvage.

Les niveaux des chimiokines MCP-1, IP-10, RANTES, IL-8, G-CSF et des cytokines pro-inflammatoires IFN-y et TNF-α sécrétées semblent indiquer que les cellules épithéliales infectées par ce virus ΔSH-rC-85473 sont en capacité de recruter les cellules immunitaires lymphocytes T, monocytes/macrophages, éosinophiles et/ou neutrophiles nécessaires à la mise en place d'une réponse immunitaire innée et adaptative de l'hôte.

De plus, le virus ΔSH-rC-85473 entraine la sécrétion d'IL-7, cytokine induisant la différenciation des lymphocytes inactifs en lymphocytes T et B actifs, et de FGF, facteur de croissance impliqué dans la réparation des tissus organiques, en quantité similaire au virus rC-85473 sauvage.

Ainsi, l'infection par le virus ΔSH-rC-85473 permet aux cellules épithéliales de mettre en place la réponse primaire de défense à l'infection, tout en étant associée à une réponse inflammatoire de l'hôte plus réduite, en comparaison avec celle induite par le virus sauvage.

Ces différences seraient ainsi en faveur de l'utilisation de virus recombinants dérivés de la souche rC-85473 comme vaccin vivant atténué, et préférentiellement de la souche atténuée ΔSH-rC-85473.

### Exemple 8 : Réplication virale et sécrétion des cytokines pro-inflammatoires IL-1β et TNF-α in vitro des virus hMPV recombinants rC-85473 sur un modèle de macrophages murins immortalisés.

Une lignée immortalisée de macrophages de souris a été utilisée pour évaluer la capacité de réplication et d'induction de cytokines pro-inflammatoires par les virus recombinants rC-85473, ΔSH-rC-85473 et ΔG-rC-85473.

Les macrophages font partie des premières cellules du système immunitaire recrutées sur le site de l'infection (dans le cas présent, sur l'épithélium respiratoire). Les macrophages participent à la réponse inflammatoire, à la réponse innée et à la stimulation de la réponse adaptative de l'hôte.

Les macrophages ont été infectés par les virus recombinants rC-85473, ΔSH-rC-85473 ou ΔG-rC-85473 et leurs surnageants cellulaires ont été récoltés chaque jour pendant 2 jours. Les résultats sont présentés en figure 8.
a) Les titres viraux mesurés en FFU/ml démontrent que le virus ΔSH-rC-85473 présente une capacité réplicative diminuée sur macrophages, par rapport au virus recombinant sauvage, alors que le virus ΔG-rC-85473 montre une réplication virale similaire au virus sauvage (figure 8A).
   Les tests statistiques Two-way ANOVA ont été réalisés pour comparer la cinétique de réplication du virus délété ΔSH-rC-85473 à celle du virus recombinant sauvage.
b) La sécrétion des cytokines pro-inflammatoires TNF-α (figure 8B) et IL-1β (figure 8C) a ensuite été mesurée dans le surnageant des macrophages infectés.

Le virus ΔSH-rC-85473 entraine un profil cytokinique significativement différent de celui du virus sauvage rC-85473 avec un pic de sécrétion plus précoce après 1 jour post-infection, en comparaison avec le virus sauvage qui est associé à un pic de sécrétion à 2 jours post-infection, concernant ces deux cytokines.

Le virus ΔG-rC-85473 induit la sécrétion de cytokine TNF-α à des niveaux comparables au virus sauvage, mais n'induit pas la sécrétion de la cytokine IL-1β durant les 2 premiers jours d'infection.

Ces résultats démontrent que le virus ΔSH-rC-85473 conserve la capacité à infecter des macrophages murins, et surtout à entrainer la sécrétion de cytokines pro-inflammatoires, malgré une cinétique modifiée.

Le virus ΔG-rC-85473 infecte efficacement les macrophages murins, mais induit plus faiblement la sécrétion de cytokines pro-inflammatoires, comme cela a été observé sur le modèle d'épithélium respiratoire humain reconstitué.

### Exemple 9 : Inflammation pulmonaire et recrutement de cellules immunitaires sur le site de l'infection in vivo induits après 5 jours d'infection de souris BALB/c par les virus hMPV recombinants rC-85473, ΔG-rC-85473 et ΔSH-rC-85473.

Des souris BALB/c ont été infectées par instillation intranasale avec :
- du milieu de culture OptiMem (mock) ou
- 5×10⁵ TCID⁵⁰ des virus recombinants rC-85473, ΔSH-rC-85473 ou ΔG-rC-85473.

Après 5 jours d'infection, 5 souris par groupe ont été euthanasiées et leurs poumons prélevés pour une analyse histo-pathologique et une mesure des scores d'inflammation résultant de l'infection (figure 9A).

Des tests statistiques ont été réalisés (two-way ANOVA) pour comparer chaque condition les unes aux autres :*p < 0.05, ** p < 0.01 and ***, p < 0.001.

La figure 9A montre que le virus sauvage rC-85473 induit une forte inflammation pulmonaire chez les souris infectées, alors que les virus ΔSH-rC-85473 et ΔG-rC-85473 entrainent une inflammation significativement plus faible que le virus sauvage, en adéquation avec la sévérité réduite de la pathologie avec les souches atténuées démontrée précédemment en figure 3.

Par ailleurs, à 5 jours post-infection, 5 souris par groupe ont été euthanasiées et leurs poumons prélevés pour la quantification absolue des cellules immunitaires lymphocytes CD4+, CD8+, neutrophiles et macrophages infiltrées dans le tissu pulmonaire à la suite de l'infection (figure 9B).

Des tests statistiques ont été réalisés (one-way ANOVA) pour comparer la condition ΔSH-rC-85473 à la condition rC-85473 : ** p < 0.01.

La figure 9B démontre que les différentes populations de cellules immunitaires (T CD4+, T CD8+, neutrophiles et macrophages) sont recrutées sur le site de l'infection après 5 jours d'inoculation des virus rC-85473 et ΔSH-rC-85473, mais en quantité plus faible pour la souche atténuée, notamment en ce qui concerne les lymphocytes CD4+.

Ainsi, il semblerait que le virus ΔSH-rC-85473 entraine une inflammation pulmonaire réduite, en cohérence avec l'atténuation de la pathologie viro-induite en modèle murin (figure 3) et la diminution de la sécrétion de cytokines par les cellules épithéliales infectées (figure 7), tout en assurant un recrutement efficace des différentes cellules immunitaires sur le site de l'infection.

Ces différents résultats (figures 7 à 9) présentent les propriétés différentes entre virus recombinants sauvage rC-85473 et délétés en ce qui concerne leurs effets physiologiques post-infection.

La souche atténuée ΔSH-rC-85473 semble particulièrement prometteuse pour le développement d'un vaccin vivant atténué, au regard de ses propriétés du point de vue de (i) la sécrétion de cytokines inflammatoires et chimiokines par les cellules épithéliales et macrophagiques, (ii) l'induction de l'inflammation pulmonaire *in vivo* et (iii) de l'infiltration *in vivo* de cellules immunitaires dans l'organe infecté. En effet, l'infection d'un hôte par cette souche atténuée est associée à la fois à une réponse immunitaire primaire efficace (équivalente à celle observée avec le virus sauvage) et à une réponse inflammatoire réduite.

### Exemple 10 : Sécrétion de cytokines/chimiokines, recrutement de cellules immunitaires sur le site de l'infection in vivo et inflammation pulmonaire en modèle murin BALB/c immunisé par les virus hMPV recombinants ΔG-C-85473 et ΔSH-C-85473, suite à un challenge infectieux.

A la suite d'une infection virale, une réponse inflammatoire et immunitaire complexe se met en place sur le site de l'infection grâce à la sécrétion de cytokines et chimiokines par les cellules épithéliales infectées (voir figure 7) et les cellules immunitaires résidentes (figure 8). En premier lieu, les cellules effectrices de la réponse innée (non-spécifique) sont recrutées et activées puis dans un deuxième temps, les cellules effectrices de la réponse adaptative (spécifique). Lorsque l'individu infecté a été préalablement immunisé ou vacciné, la réponse adaptative est d'autant plus rapide, importante et spécifique que la réponse mémoire est efficace. C'est la caractérisation de cette réponse immunitaire, dite secondaire, qui est présentée dans cet exemple.

Des souris BALB/c ont été immunisées par instillation intranasale avec du milieu de culture OptiMem (témoin négatif, « mock ») ou 5×10⁵ TCID⁵⁰ des virus recombinants rC-85473, ΔSH-C-85473 ou ΔG-C-85473.

21 jours après l'immunisation, les souris ont été infectées (challenge infectieux) par instillation intranasale d'une dose létale (1×10⁶ TCID⁵⁰) du virus rC-85473 sauvage. Le protocole d'immunisation et du challenge infectieux est présenté en figure 4A.

1 ou 5 jours après le challenge infectieux (soit aux jours 22 ou 26 post-immunisation respectivement), 3 souris de chaque groupe ont été euthanasiées pour quantifier une sélection de cytokines et chimiokines sécrétées dans le tissu pulmonaire : les résultats sont présentés en figures 10A, 10B et 10C. Ces résultats démontrent l'induction d'une réponse immunitaire locale différente en fonction des virus recombinants rC-85473 utilisés pour l'immunisation des souris BALB/c :
- Les groupes immunisés par les virus ΔG-C-85473 et ΔSH-C-85473 montrent une sécrétion de la cytokine pro-inflammatoire IL-6 et de la chimiokine MCP-1 plus forte que le groupe immunisé par le virus WT rC-85473, rapidement 1 jour après le challenge infectieux.
- De plus, le groupe immunisé par le virus ΔSH-C-85473 montre aussi une augmentation significative de la sécrétion de la cytokine anti-inflammatoire IL-10 et des chimiokines G-CSF et MIP-1β, par rapport au groupe immunisé par le virus WT et/ou par le virus ΔG-C-85473

Après 5 jours, toutes les cytokines/chimiokines testées sont en forte diminution par rapport aux niveaux mesurés au jour 1 post-challenge, ce qui démontre une résorption de la réponse cytokinique et inflammatoire induite par le challenge.

Ces résultats suggèrent que l'immunisation par les virus atténués ΔG-C-85473, et plus particulièrement ΔSH-C-85473, entraine la mise en place localement d'une réponse cytokinique différente à celle induite par l'immunisation par le virus WT non atténué correspondant.

Pour évaluer le recrutement des cellules de l'immunité sur le site de l'infection, la quantité des principales populations de cellules immunitaires qui s'infiltrent dans le tissu pulmonaire pour lutter contre l'infection a été mesurée ; les résultats sont présentés dans les figures 10D, 10E et 10F.

Ainsi, le recrutement des lymphocytes T (CD8+ cytotoxiques et CD4+ *Helpers*) et des lymphocytes B est significativement augmenté suite à l'immunisation avec le virus ΔSH-C-85473, par rapport au groupe immunisé par le virus WT rC-85473, et par rapport au groupe immunisé par le virus ΔG-C-85473 (différence significative uniquement pour les lymphocytes B, figure 10E), 5 jours après le challenge infectieux.

Les populations de macrophages et neutrophiles recrutées ne montrent pas de différences significatives entre les différents virus testés (figure 10F).

Le groupe de souris immunisées par le virus ΔG-C-85473 montre un profil de recrutement cellulaire similaire à celui du groupe immunisé par le virus WT, ce qui suggère que le virus ΔG-C-85473 est aussi efficace pour l'induction de la réponse immunitaire qu'un virus sauvage (WT). Au contraire, lorsque les souris ont préalablement été immunisées avec le virus ΔSH-C-85473, la réponse immunitaire adaptative en réponse au challenge infectieux est plus importante, tout en restant équilibrée, alors que la réponse innée ne semble pas affectée, ce qui suggère que le virus ΔSH-C-85473 induirait une réponse immunitaire mémoire plus efficace.

Finalement, 5 jours après le challenge, 2 souris par groupe ont été euthanasiées pour mesurer les scores d'inflammation pulmonaire résultant du challenge de chaque groupe immunisé et non-immunisé « mock » ; les résultats sont présentés en figure 10G.

Tel qu'attendu avec l'utilisation d'un inoculum létal de virus WT rC-85473, qui correspond à une mortalité de 50% de l'effectif [voir figure 4C], le score cumulatif d'inflammation du groupe mock-immunisé est très élevé. Comme précédemment, les scores d'inflammation des groupes immunisés par le virus WT rC-85473 ou ΔG-C-85473 sont très comparables entre eux, et faiblement diminué par rapport au groupe mock-immunisé bien que les souris de ces groupes ne démontrent aucune mortalité [Figure 4C].

Les résultats de la figure 10 montrent que les souris immunisées par le virus ΔSH-C-85473 mettent en place une réponse inflammatoire très réduite suite au challenge infectieux, ce qui suggère que les atteintes pulmonaires seraient réduites dans ce groupe. De même les fonctionnalités pulmonaires seraient conservées au cours de l'infection.

Ainsi, le virus atténué ΔSH-C-85473 pourrait permettre un meilleur recrutement pulmonaire des cellulaires immunitaires, en quantité et en qualité, en réponse à une infection secondaire, et ainsi contribuer à limiter la physiopathologie et l'inflammation pulmonaire de l'hôte infecté. Cette réponse de l'hôte plus efficace pourrait aussi témoigner d'une meilleure réponse immunitaire mémoire contre le virus HMPV.

En conclusion, ce virus atténué ΔSH-C-85473 présente les caractéristiques suivantes :
(i) il induit de plus fortes quantités de cytokines pro-inflammatoires, anti-inflammatoires et chimiokines que les autres virus testés ;
(ii) ceci permettant un recrutement plus important, mais équilibré, des cellules immunitaires (lymphocytes T CD8+/CD4+ et lymphocytes B) sur le site de l'infection,
(iii) tout en générant une inflammation pulmonaire limitée.

**Tableau 8. Récapitulatif des séquences présentées dans le séquence listing**

| | Description |
|---|---|
| SEQ ID NO. 1 | Séquence génomique complète de la souche sauvage de hMPV C-85473 |
| SEQ ID NO. 2 | Séquence complète du virus HMPV recombinant ΔSH-rC-85473 |
| SEQ ID NO. 3 | Séquence complète du virus HMPV recombinant ΔG-rC-85473 |
| SEQ ID NO. 4 | Séquence complète du virus HMPV recombinant sauvage rC-85473 couplée à la séquence de la GFP |
| SEQ ID NO. 5 | Séquence complète du virus HMPV recombinant ΔSH-rC-85473 couplée à la séquence de la GFP |
| SEQ ID NO. 6 | Séquence complète du virus HMPV recombinant ΔG-rC-85473 couplée à la séquence de la GFP |
| SEQ ID NO. 7 | Séquence complète du génome HMPV sauvage CAN98-75 |
| SEQ ID NO. 8 | Séquence complète du virus HMPV recombinant sauvage CAN98-75 couplée à la séquence de la GFP |

### REFERENCES

### Brevets

WO 2005/014626
US 8,841,433

### Références bibliographiques par ordre de citation dans le texte

van den Hoogen BG, de Jong JC, Groen J, Kuiken T, de Groot R, Fouchier RA, et al. A newly discovered human pneumovirus isolated from young children with respiratory tract disease. Nat Med. 2001;7(6):719-24
Peret TC, Boivin G, Li Y, Couillard M, Humphrey C, Osterhaus AD, Erdman DD, Anderson LJ. Characterization of human metapneumoviruses isolated from patients in North America. J Infect Dis. 2002 Jun 1;185(11):1660-3.
Mazur NI, Higgins D, Nunes MC, Melero JA, LangedijkAC, Horsley N, Buchholz UJ, Openshaw PJ, McLellan JS, Englund JA, Mejias A, Karron RA, Simôes EA, Knezevic I, Ramilo O, Piedra PA, Chu HY, Falsey AR, Nair H, Kragten-Tabatabaie L, Greenough A, Baraldi E, Papadopoulos NG, Vekemans J, Polack FP, Powell M, Satav A, Walsh EE, Stein RT, Graham BS, Bont LJ; Respiratory Syncytial Virus Network (ReSViNET) Foundation. The respiratory syncytial virus vaccine landscape: lessons from the graveyard and promising candidates. Lancet Infect Dis. 2018 Jun 15. pii: S1473-3099(18)30292-5.
Herfst S, de Graaf M, Schrauwen EJ, Sprong L, Hussain K, van den Hoogen BG, Osterhaus AD, Fouchier RA. Génération of temperature-sensitive human metapneumovirus strains that provide protective immunity in hamsters. J Gen Virol. 2008 Jul;89(Pt 7):1553-62.
Wei Y, Zhang Y, Cai H, Mirza AM, lorio RM, Peeples ME, Niewiesk S, Li J. Roles of the putative integrin-binding motif of the human metapneumovirus fusion (f) protein in cell-cell fusion, viral infectivity, and pathogenesis. J Virol. 2014 Apr;88(8):4338-52.
Yu CM, Li RP, Chen X, Liu P, Zhao XD. Replication and pathogenicity of attenuated human metapneumovirus F mutants in severe combined immunodeficiency mice. Vaccine. 2012 Jan 5;30(2):231-6.
Liu P, Shu Z, Qin X, Dou Y, Zhao Y, Zhao X. A live attenuated human metapneumovirus vaccine strain provides complété protection against homologous viral infection and cross-protection against heterologous viral infection in BALB/c mice. Clin Vaccine Immunol. 2013 Aug;20(8):1246-54.
Zhang Y, Wei Y, Zhang X, Cai H, Niewiesk S, Li J. Rational design of human metapneumovirus live attenuated vaccine candidates by inhibiting viral mRNA cap methyltransferase. J Virol. 2014 Oct;88(19):11411-29
Biacchesi S, Skiadopoulos MH, Tran KC, Murphy BR, Collins PL, Buchholz UJ. Recovery of human metapneumovirus from cDNA: optimization of growth in vitro and expression of additional genes. Virology. 2004;321(2):247-59
Biacchesi S, Skiadopoulos MH, Yang L, Lamirande EW, Tran KC, Murphy BR, Collins PL, Buchholz UJ. Recombinant human Metapneumovirus lacking the small hydrophobie SH and/or attachment G glycoprotein: délétion of G yields a promising vaccine candidate. Journal of Virology. 2004 Dec;78(23):12877-87.
Biacchesi S, Pham QN, Skiadopoulos MH, Murphy BR, Collins PL, Buchholz UJ. Infection of nonhuman primates with recombinant human metapneumovirus lacking the SH, G, or M2-2 protein categorizes each as a nonessential accessory protein and identifies vaccine candidates. Journal of virology. 2005;79(19):12608-13.
Buchholz UJ, Biacchesi S, Pham QN, Tran KC, Yang L, Luongo CL, Skiadopoulos MH, Murphy BR, Collins PL. Délétion of M2 gene open reading frames 1 and 2 of human metapneumovirus: effects on RNA synthesis, atténuation, and immunogenicity. J Virol. 2005 Jun;79(11):6588-97.
Schickli JH, Kaur J, Macphail M, Guzzetta JM, Spaete RR, Tang RS. Délétion of human metapneumovirus M2-2 increases mutation frequency and attenuates growth in hamsters. Virol J. 2008 Jun 3;5:69.
Pham QN, Biacchesi S, Skiadopoulos MH, Murphy BR, Collins PL, Buchholz UJ. Chimeric recombinant human metapneumoviruses with the nucleoprotein or phosphoprotein open reading frame replaced by that of avian metapneumovirus exhibit improved growth in vitro and atténuation in vivo. J Virol. 2005 Dec;79(24):15114-22.
Tang RS, Schickli JH, MacPhail M, Fernandes F, Bicha L, Spaete J, Fouchier RA, Osterhaus AD, Spaete R, Haller AA. Effects of human metapneumovirus and respiratory syncytial virus antigen insertion in two 3' proximal genome positions of bovine/human parainfluenza virus type 3 on virus réplication and immunogenicity. J Virol. 2003 Oct;77(20):10819-28.
Tang RS, Mahmood K, Macphail M, Guzzetta JM, Haller AA, Liu H, Kaur J, Lawlor HA, Stillman EA, Schickli JH, Fouchier RA, Osterhaus AD, Spaete RR. A host-range restricted parainfluenza virus type 3 (PIV3) expressing the human metapneumovirus(hMPV) fusion protein elicits protective immunity in African green monkeys. Vaccine. 2005 Feb 25;23(14):1657-67.
Russell CJ, Jones BG, Sealy RE, Surman SL, Mason JN, Hayden RT, Tripp RA, Takimoto T, Hurwitz JL. A Sendai virus recombinant vaccine expressing a gene for truncated human metapneumovirus (HMPV) fusion protein protects cotton rats from hMPV challenge. Virology. 2017 Sep;509:60-66.
Huck B, Scharf G, Neumann-Haefelin D, Puppe W, Weigl J, Falcone V. Novel human metapneumovirus sublineage. Emerg Infect Dis. 2006 Jan;12(1):147-50.
Aerts L, Rhéaume C, Carbonneau J, Lavigne S, Couture C, Hamelin ME, Boivin G. Adjuvant effect of the human metapneumovirus (HMPV) matrix protein in HMPV subunit vaccines. J Gen Virol. 2015 Apr;96(Pt 4):767-74.
Dubois J, Cavanagh MH, Terrier O, Hamelin ME, Lina B, Shi R, et al. Mutations in the fusion protein heptad repeat domains of human metapneumovirus impact on the formation of syncytia. The Journal of general virology. 2017;98(6):1174-80.
Aerts L, Cavanagh MH, Dubois J, Carbonneau J, Rheaume C, Lavigne S, et al. Effect of in vitro syncytium formation on the severity of human metapneumovirus disease in a murine model. PloS one. 2015;10(3):e0120283.
Hamelin ME, Gagnon C, Prince GA, Kiener P, Suzich J, Ulbrandt N, Boivin G. Prophylactic and therapeutic benefits of a monoclonal antibody against the fusion protein of human metapneumovirus in a mouse model. Antiviral Res. 2010 Oct;88(1):31-7.
Palavecino CE, Cespedes PF, Lay MK, Riedel CA, Kalergis AM, Bueno SM. Understanding Lung Immunopathology Caused by the Human Metapneumovirus: Implications for Rational Vaccine Design. Crit Rev Immunol. 2015;35(3):185-202. Review.
Hamelin ME, Couture C, Sackett MK, Boivin G. Enhanced lung disease and Th2 response following human metapneumovirus infection in mice immunized with the inactivated virus. J Gen Virol. 2007 Dec;88(Pt 12):3391-400. PubMed PMID: 18024909.

## Revendications

1. Souche virale atténuée dérivée de la souche rC-85473 de metapneumovirus humain comprenant la séquence génomique représentée par la séquence SEQ ID NO. 1, ladite souche atténuée ayant été modifiée génétiquement exclusivement pour atténuer sa virulence, **caractérisée en ce que** les modifications de la séquence SEQ ID NO.1 consistent en l'inactivation du gène codant pour la protéine SH et/ou l'inactivation du gène codant pour la protéine G.

2. Souche virale atténuée selon la revendication 1, **caractérisée en ce que** ladite souche comprend la séquence SEQ ID NO.2.

3. Souche virale atténuée selon la revendication 1, **caractérisée en ce que** ladite souche comprend la séquence SEQ ID NO.3.

4. Souche virale atténuée selon la revendication 1, **caractérisée en ce que** les modifications de la séquence SEQ ID NO.1 consistent en l'inactivation des deux gènes codant pour la protéine G et la protéine SH.

5. Souche virale atténuée selon l'une des revendications 1 à 4, dont la séquence comprend au moins un gène exogène, en particulier un gène codant pour un antigène viral.

6. Utilisation in vitro d'une souche virale atténuée selon la revendication 5, en tant que vecteur d'expression d'au moins un gène exogène dans des cellules cibles du metapneumovirus humain.

7. Souche virale atténuée selon l'une des revendications 1 à 5, pour son utilisation en tant que médicament.

8. Souche virale atténuée selon l'une des revendications 1 à 5, pour son utilisation pour prévenir et/ou traiter les infections par des virus de la famille des Pneumoviridae.

9. Souche virale atténuée pour son utilisation selon la revendication 8, pour prévenir et/ou traiter les infections par des metapneumovirus humains.

10. Souche virale atténuée pour son utilisation selon la revendication 8, pour prévenir et/ou traiter les infections par des orthopneumovirus, tel que le virus respiratoire syncytial humain.

11. Souche virale atténuée selon la revendication 5 pour son utilisation pour prévenir et/ou traiter les infections par des virus dont au moins un antigène viral est exprimé par ladite souche virale atténuée.

12. Composition vaccinale comprenant, dans un véhicule pharmaceutiquement acceptable, au moins une souche virale atténuée selon l'une des revendications 1 à 5, et optionnellement un adjuvant.

13. Composition vaccinale selon la revendication 12, **caractérisée en ce qu'**elle est sous une forme galénique destinée à une administration par inhalation.

## Patentansprüche

1. Abgeschwächter Virusstamm, der von dem humanen Metapneumovirus-Stamm rC-85473 abgeleitet ist, der die durch die Sequenz SEQ ID Nr. 1 dargestellte Genomsequenz umfasst, wobei der abgeschwächte Stamm genetisch modifiziert wurde, um ausschließlich seine Virulenz abzuschwächen, **dadurch gekennzeichnet, dass** die Modifikationen der Sequenz SEQ ID Nr. 1 aus der Inaktivierung des für das SH-Protein kodierenden Gens und/oder die Inaktivierung des für das G-Protein kodierenden Gens bestehen.

2. Abgeschwächter Virusstamm nach Anspruch 1, **dadurch gekennzeichnet, dass** der Stamm die Sequenz SEQ ID Nr. 2 umfasst.

3. Abgeschwächter Virusstamm nach Anspruch 1, **dadurch gekennzeichnet, dass** der Stamm die Sequenz SEQ ID Nr. 3 umfasst.

4. Abgeschwächter Virusstamm nach Anspruch 1, **dadurch gekennzeichnet, dass** die Modifikationen der Sequenz SEQ ID Nr. 1 aus der Inaktivierung der beiden Gene, die für das G-Protein und das SH-Protein kodieren, bestehen.

5. Abgeschwächter Virusstamm nach einem der Ansprüche 1 bis 4, dessen Sequenz mindestens ein exogenes Gen umfasst, insbesondere ein Gen, das für ein virales Antigen kodiert.

6. In-vitro-Verwendung eines abgeschwächten Virusstamms nach Anspruch 5 als Vektor für die Expression mindestens eines exogenen Gens in Zielzellen des humanen Metapneumovirus.

7. Abgeschwächter Virusstamm nach einem der Ansprüche 1 bis 5 für seine Verwendung als Arzneimittel.

8. Abgeschwächter Virusstamm nach einem der Ansprüche 1 bis 5 für seine Verwendung zur Vorbeugung und/oder Behandlung von Infektionen durch Viren aus der Familie der Pneumoviridae.

9. Abgeschwächter Virusstamm für seine Verwendung nach Anspruch 8 zur Vorbeugung und/oder Behandlung von Infektionen durch humane Metapneumoviren.

10. Abgeschwächter Virusstamm für seine Verwendung nach Anspruch 8 zur Vorbeugung und/oder Behandlung von Infektionen durch Orthopneumoviren wie das humane Respiratory Syncytial Virus.

11. Abgeschwächter Virusstamm nach Anspruch 5 für seine Verwendung zur Vorbeugung und/oder Behandlung von Infektionen durch Viren, von denen mindestens ein virales Antigen durch den abgeschwächten Virusstamm exprimiert wird.

12. Impfstoffzusammensetzung, die in einem pharmazeutisch akzeptablen Träger mindestens einen abgeschwächten Virusstamm nach einem der Ansprüche 1 bis 5 und optional ein Adjuvans enthält.

13. Impfstoffzusammensetzung nach Anspruch 12, **dadurch gekennzeichnet, dass** sie in einer Darreichungsform für die Verabreichung durch Inhalation vorliegt.

## Claims

1. An attenuated viral strain derived from human metapneumovirus strain rC-85473 comprising the genomic sequence represented by the sequence SEQ ID NO. 1, said attenuated strain having been genetically modified exclusively to attenuate its virulence, **characterized in that** the modifications of the sequence SEQ ID NO.1 consist of the inactivation of the coding gene for the SH protein and/or the inactivation of the coding gene for G protein.

2. The attenuated viral strain according to claim 1, **characterized in that** said strain comprises the sequence SEQ ID NO.2.

3. The attenuated viral strain according to claim 1, **characterized in that** said strain comprises the sequence SEQ ID NO.3.

4. The attenuated viral strain according to claim 1, **characterized in that** the modifications of the sequence SEQ ID NO.1 consist of the inactivation of the two coding genes for the G protein and the SH protein.

5. The attenuated viral strain according to one of claims 1 to 4, the sequence of which comprises at least one exogenous gene, in particular a coding gene for a viral antigen.

6. An in vitro use of an attenuated viral strain according to claim 5, as a vector for expressing at least one exogenous gene in target cells of the human metapneumovirus.

7. The attenuated viral strain according to one of claims 1 to 5, for its use as a medicine.

8. The attenuated viral strain according to one of claims 1 to 5, for its use to prevent and/or treat infections by viruses of the Pneumoviridae family.

9. The attenuated viral strain for its use according to claim 8, to prevent and/or treat infections by human metapneumoviruses.

10. The attenuated viral strain for its use according to claim 8, to prevent and/or treat infections by orthopneumoviruses, such as human respiratory syncytial virus.

11. The attenuated viral strain according to claim 5 for its use to prevent and/or treat infections by viruses of which at least one viral antigen is expressed by said attenuated viral strain.

12. A vaccine composition comprising, in a pharmaceutically acceptable vehicle, at least one attenuated viral strain according to one of claims 1 to 5, and optionally an adjuvant.

13. The vaccine composition according to claim 12, **characterized in that** it is in a galenic form intended for administration by inhalation.
